# EUROPEAN PATENT APPLICATION

(11) **EP 3 594 226 A1**
(43) Date of publication of application: **15.01.2020**
(21) Application number: 18183493.8
(22) Date of filing: 13.07.2018
(51) Int. Cl.: C07K 14/435

(54) **ANT VENOM PEPTIDOME**

(71) Applicant: Institut National Universitaire Champollion, 81012 Albi, cedex 09 (FR); Institut National de la Santé et de la Recherche Médicale (INSERM), 75013 Paris (FR); Universite de Rouen Normandie, 76821 Mont Saint Aignan Cedex (FR)
(72) Inventor: TREILHOU, Michel, 81380 Lescure d Albigeois (FR); BONNAFE, Elsa, 81000 Albi (FR); TOUCHARD, Axel, 81000 Albi (FR); LEPRINCE, Jérôme, 76130 Mont Saint Aignan (FR)
(74) Representative: Agasse, Stéphane

(57) **Abstract**

The present invention deals with isolated venom peptide precursors comprising a prepropeptide sequence selected from the group consisting of SEQ ID No. 1, SEQ ID No. 2, SEQ ID No. 3, SEQ ID No. 4, SEQ ID No. 95 or SEQ ID No. 96 and a mature peptide

## Description

Venoms are sophisticated biochemical cocktails comprising a large array of bioactive compounds. Peptides are the predominant class of component of most arthropod venoms and are currently under investigation for their potential as therapeutic lead compounds, molecular probes or insect-selective bio-pesticides. Indeed, the large molecular diversity of peptide toxins is straight linked to a broad array of biological activities and pharmacological targets. Indubitably, the extensive exploration of venoms from both well- and less-studied venomous taxa using cutting-edge technologies will lead to valuable discoveries.

In contrast to cone snails, snakes, scorpions and spiders, the venom composition of a wide range of venomous animals, mostly insects, has been less studied. Investigation of such neglected organisms is important to understand the whole diversity of venomous systems among the animal kingdom and to give novel insights into the evolution of venom toxins. Ants (Hymenoptera: Formicidae) belong to a dominant, diverse but also jilted group of venomous arthropods. Although mass spectrometry-based studies of a variety of ant venoms have revealed the presence of thousands peptides per species among which some hold novel structural features less than 100 venom peptides have been characterized from a limited number of ant species. The scarcity of studies on ant venom peptides is due to the small size of specimen and the difficulties in collecting their venoms. However, the advances in "omics" technologies allow now to overcome these limitations and explore such venom compositions in detail. Recent transcriptomic studies (RNA-Seq) of venom glands have started to uncover the genes encoding the molecular diversity of the ant venom peptides.

Transcriptomic based-studies, particularly those using next generation sequencing platforms, provide a high sensitive and high-throughput technique for the exploration of venom composition at the RNA level. This approach yields a set of putative precursor sequences from which the mature peptides are subsequently predicted. Nevertheless, the shortness of open reading frames (ORFs) encoding peptides complicates their identification, and prediction of cleavage maturation sites can be difficult due to their non-conventional nature. Furthermore, transcriptomic RNA-Seq analyses may generate numerous inherent errors within the transcriptome data set due to the *de novo* assembly of short reads into contigs leading to shifted or incomplete ORFs. Proteomic analyses, typically liquid chromatography coupled to mass spectrometry (LC-MS), are often used in combination of transcriptomic data and provide a direct examination of venom peptides in order to confirm and/or identify predicted sequences from transcriptomics data. Additionally, mass spectrometry allows the identification of the post-translational modifications (PTMs) that are often observed in mature venom peptides. Such integrated venomics approach has revolutionized the field of toxinology in well-studied venomous organisms generating an accurate picture of their venom composition. Venomics studies has also begun to shed light on the molecular diversity of venom from a range of poorly studied venomous taxa and has been applied very recently to only one ant venom. Indeed, most of the previous studies about ant venoms were conducted on isolated peptides and there is actually no comprehensive investigation on the repertoire of peptide toxins found in the venom of a single species. The aim of this work was to uncover the whole molecular diversity of peptides expressed by the venom glands of the ant *Tetramorium bicarinatum.*

In order to gain further insight into the venom peptide arsenal of *T. bicarinatum,* We identified a total of 37 peptide toxin precursors. Further clustering analyses permitted us to classify these precursors into three superfamilies.

The first objet of the present invention relates to an isolated venom peptide precursor comprising a prepropeptide sequence selected from the group consisting of SEQ ID No. 95 and more preferably SEQ ID No. 1, SEQ ID No. 96 and more preferably SEQ ID No 2; SEQ ID No. 3 or SEQ ID No.4 and a mature peptide.

In a specific aspects, the isolated venom peptide precursor
- of SEQ ID No. 1 or of SEQ ID No. 95 comprises a the cleavage motif of SEQ ID No. 11 between the prepropeptide and the mature peptide.
- of SEQ ID No. 2 or of SEQ ID No. 96 comprises a the cleavage motif chosen among SEQ ID No. 12 to SEQ ID No. 15.
- of SEQ ID No. 3 or of SEQ ID No. 4 comprises a the cleavage motif chosen among among GEA, SEA, TEA, TEF or TEG.

According to a more specific aspect of the invention the venom peptide precursor belongs to superfamily A and is chosen among SEQ ID No. 16 to SEQ ID No. 30.

Those peptides precursors belonging to superfamily A are pilosulin precursors.

According to a more specific aspect of the invention the venom peptide precursor belongs to superfamily B and is chosen among SEQ ID No. 31 to SEQ ID No. 38.

Those peptides precursors belonging to superfamily B are precursors of antimicrobial peptides.

According to a more specific aspect of the invention the venom peptide precursor belongs to superfamily C and is chosen among SEQ ID No. 39 to SEQ ID No. 50.

Those peptides precursors belonging to superfamily C are secapin precursors.

In a particular aspect of the invention, the mature venom peptide deriving from an isolated venom peptide precursors described above is chosen among SEQ ID No.51 to SEQ ID No. 88.

In another aspect, the invention deals with the nucleic acids encoding anyone of the peptide of SEQ ID No.31 to SEQ ID No. 88.

### 1. MATERIALS and METHODS

### 1.1 Ants and venom sample preparation

Two colonies of *T. bicarinatum* were collected by Dr. Jérôme Orivel in Itabuna, Bahia state, Brazil in 2000 and were harbored in our laboratory. The colonies are maintained at 25°C and fed three times a week with fresh mealworms and an aqueous honey solution (1:1 v/v). Ant venom sacs were dissected and pooled in water with 1% formic acid (v/v) and membranes were disrupted by ultrasonic waves for 2 min. Then, samples were centrifuged for 5 min at 14,400 rpm, the supernatant was collected and dried with speed vacuum prior to storage at -20°C until use. Venom samples of 100 venom sacs were mixed to conduct all the proteomic analyses.

### 1.2 Mass spectrometry analysis and venom fractionation

Preliminary LC-MS analysis of the crude venom was carried out on the LCQ-Ion trap Surveyor equipped with an ESI-LC system Advantage (ThermoFisher Scientific, France). Peptides were separated using a Luna-C18 column (5 µm; 2 × 150 mm; Phenomenex, France). The mobile phase was a gradient prepared from 0.1% aqueous formic acid (solvent A) and 0.1% formic acid in acetonitrile (solvent B). Peptides were eluted using a linear gradient from 0 to 50% B over 45 min, from 50% to 100% B over 10 minutes and then held for 5 min at 200 µL.min-1 flow rate. The electrospray ionization mass spectrometry detection was performed in positive mode with the following optimized parameters: capillary temperature set at 300°C, spray voltage was at 4.5kV, sheath gas and auxiliary gas set at 50 and 10 psi, respectively. The acquisition range was from 100 to 2000 m/z. The fractions were collected each minute and dried for further mass spectrometry-based *de novo* sequencing or Edman degradation.

### 1.3 De novo Orbitrap mass spectrometry-based sequencing

Each venom fraction was resuspended in d.i. water and subjected to *de novo* sequencing using a LTQ-Orbitrap Elite coupled to an Easy nLC II system (both from Thermo Scientific). Samples were injected onto an enrichment column (C18 PepMap100, ThermoFisher Scientific). The separation was achieved with an analytical column needle (NTCC-360/100-5-153, NikkyoTechnos). The mobile phase consisted of H2O/FA 0.1% (buffer A) and ACN/FA 0.1% (buffer B). Tryptic peptides were eluted at a flow rate of 300 nL.min⁻¹, using a linear gradient from 5 to 45% B over 109 min. The mass spectrometer was operated in positive ionization mode with capillary voltage and source temperature set at 1.5 kV and 275 °C, respectively. The samples were analyzed using both HCD (Higher-energy Collision Dissociation) and CID (collision induced dissociation) methods. The first scan (MS spectra) was recorded in the Orbitrap analyzer (R = 60,000) with the mass range m/z 400-1800. Then, the 10 or 20 most intense ions were selected for MS2 experiments in HCD or CID modes, respectively. The resolution for MS2 experiment in the case of HCD mode was 30,000. Only di- and tri-charged ions were selected, others charge states were excluded for MS2 experiments. Dynamic exclusion of already fragmented precursor ions was applied for 30 s, with a repeat count of 1, a repeat duration of 30 s and an exclusion mass width of ±10 ppm. The precursor isolation width was 2 m/z. Fragmentation occurred in the linear ion trap analyzer with a normalized collision energy of 35%. All measurements in the Orbitrap analyzer were performed with on-the-fly internal recalibration (lock mass) at m/z 445.12002 (polydimethylcyclosiloxane). Peptide *de novo* sequencing was performed by using the PEAKS studio 7.5 proteomics workbench (Bioinformatics Solutions Inc.) with the following specific parameters: enzyme, none; variable modifications, oxidation (M), amidation, deamidation (NQ), pyro-glu from E and Q; monoisotopic; mass tolerance for precursor ions, 20 ppm; mass tolerance for fragment ions, 50 ppm; MS scan mode, orbitrap; MS/MS scan mode, linear ion trap. High confident PEAKS de novo sequencing result was filtered by average local confidence score (ALC ≥ 70%).

### 1.4 Edman sequencing

The primary structures of the peptides were determined by automated Edman degradation. Venom fractions containing isolated peptide were resuspended in an acetonitrile, trifluoroacetic acid, water solution (30/1/69 v/v/v), and were loaded onto a precycled Biobrene Plus-coated glass filter. The N-terminal sequences were then determined by introducing the filter disc into a Procise P494 automated protein sequenator (Applied Biosystems, Foster City, CA) and runs of Edman degradation were carried out.

### 1.5 Glycosylation study

Synthetic Fmoc-Thr(*O*-GalNAc)-OH or *O*-GlcNAc (2 pmol) and crude venom were respectively analyzed with a nano-LC1200 system coupled to a Q-TOF 6550 mass spectrometer equipped with a nanospray source and an HPLC-chip cube interface (Agilent Technologies, Santa Clara, CA). For crude venom, a 26-min linear gradient (3-75% acetonitrile in 0.1% formic acid), at a flow rate of 350 nL.min⁻¹, was used to separate peptides on polaris-HR-Chip C18 column (150 mm long x 75 µm inner diameter). Full autoMS1 scans from 290 to 1700 m/z and autoMS2 from 59 to 1700 m/z were recorded. In every cycle, a maximum of 5 precursors were isolated and fragmented into the collision cell with a fixed collision energy (20, 25, 27, 30 and 35 eV). Each experiment was set to use an inclusion list for the single charged of the synthetic O-Thr and the 2+ and 3+ of each specific peptide. Active exclusion of these precursors was enabled after 1 spectrum within 0.13 min, and the absolute threshold for precursor selection was set to 1000 counts (relative threshold 0.001%).

### 1.6 Direct sequencing of predicted cDNA

To confirm or to complete cDNA sequences and to verify that the genes encoding the peptides are expressed in venom glands, we have isolated cDNA encoding peptides by a direct molecular approach. Briefly, total RNAs from 200 venom glands were extracted in TRIzol reagent (Invitrogen, Carlsbad, CA) according to the manufacturer's protocol. Contaminating genomic DNA was removed using DNA-free kit (Applied Biosystem) following the manufacturer's instruction. RNA quantity was evaluated using a nanodrop (Nanodrop 2000, ThermoFisher Scientific). To confirm complete cDNA sequence, cDNA was synthesized from 100 ng of total RNA by M-MLV reverse transcriptase (Invitrogen) at 37°C for 50 minutes with random hexamers following the protocol of the manufacturer. To complete cDNA sequences, cDNA was synthetized from 500 ng of total RNA with M-MLV (Invitrogen) and 5'RACE adapter from the FirstChoice RLM-RACE kit (Invitrogen) following the protocol of the manufacturer. PCR experiments were performed by using specifics reverse primers designed manually with predicted sequences and 5'inner and outer adapter primers from the FirstChoice RLM-RACE kit in order to obtained the complete 5'cDNA part of sequence encoding the prepro-peptide. Then, PCR products have been either cleaned using PureLink PCR purification kit (Invitrogen) or purified from agarose gel, when several products have been amplified, with PureLink™ Quick Gel Extraction Kit (Invitrogen) and sequenced on Get-TQ platform (Purpan, Toulouse, France). Complete ORFs have been determined using translate program, subsequently aligned using seaview plateform with muscle program and formatted using BOXSHADE 3.3.1 program.

### 1.7 Bioinformatic tools

Peptide sequences obtained by mass spectrometry or prepro-peptide sequences obtained from previous studies ^{11, 15-16} were submitted on *National Center for Biotechnology Information site* (ncbi, http://www.ncbi.nlm.nih.gov/) using blast program against the Transcriptome Shotgun Assembly (TSA) from *T. bicarinatum* (accession number GASM00000000.1) with algorithm parameters adapted to short sequences tblastn searches. Putative complete ORFs were then obtained on ExPASy Bioinformatics Resource Portal (http://www.expasy.org/) using translate program. When predicted ORFs were complete, the prepro-peptide sequences were deduced and used to search new unknown peptide sequences with blast program on ncbi website. Generally, ORFs were incomplete or shifted. The contigs containing partial or shifted ORFs were then reassembled using blast2q and clustal Omega program. The mass of mature peptide sequence obtained from these different approaches were systematically verified using peptide mass program and compared to those obtained by mass spectrometry. Secondary structure predictions have been done with EMBOSS 6.3.1: Garnier program. Signal sequence and transmembrane domain have been predicted with phobius program available on http://phobius.sbc.su.se/. Phylogenetics trees have been generated with MEGA software (megacc-7.0.26-1.x86 64) with Minimum Evolution (ME) or Maximum of Likelihood (ML) statistical methods, p-distance or Poisson model and edited with Seaview plateform (1:4.4.2-1)

### 2. RESULTS

### 2.1 Venom mass fingerprinting of T. bicarinatum venom

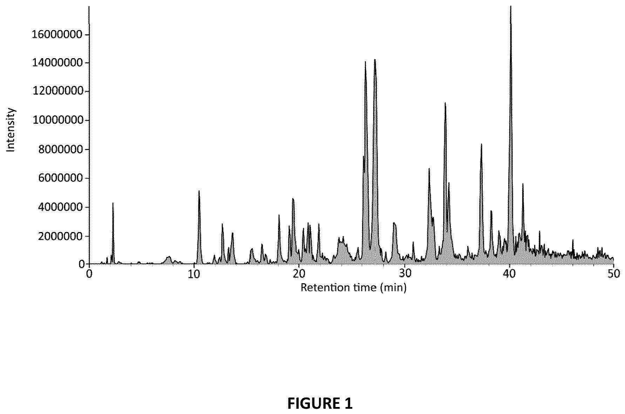

A first list of the most abundant peptides in the venom of *T. bicarinatum* was established through an initial LC-MS analysis on a LCQ-ion trap mass spectrometer. Prior to the determination of their sequences, all the peptides detected were tentatively named according to the initials of the genus and species followed by their molecular weight (*i.e.* Tb-XXXX) as described by Johnson et al. (A biochemical characterization of the major peptides from the venom of the giant Neotropical hunting ant Dinoponera australis. Toxicon 2010, 55 (4), 702-710). The fully sequenced peptides have been subsequently renamed in accordance to the nomenclature developed for venom peptides. LC-MS analysis revealed 48 masses corresponding to peptides in the venom of *T. bicarinatum* (Figure 1 and Table 1). As expected for an ant venom, all the peptides were relatively small and exhibited molecular weight ranging from 939 to 4018 Da corresponding approximately to 8-36 residue-long peptides. These peptides eluted at retention times between 2.27 and 40.1 min equivalent to 6 and 35% of acetonitrile in the mobile phase. The relative abundance of the 48 peptides was assessed and reported in Table 1. The ten most abundant peptides i.e. Tb-2212 (bicarinalin), Tb-1571 (P17), Tb-2485, Tb-3325, Tb-1651, Tb-2638, Tb-4018, Tb-3882, Tb-1207, and Tb-1934 appeared to account for more than 67% of the venom peptide content. We have analyzed its peptide content using a cutting-edge orbitrap mass spectrometer. Analysis resulted in a total number of 2,853 detected peptides in the 1000-3000 Da mass range for 67% of them (data not shown).

Molecular weight of the 48 previously detected peptides has been accurately adjusted in Table 1.

Then, we focused on the sequence identification of the venom peptides using a combination of direct and *de novo* sequencing. First, the venom was separated by reversed-phase HPLC using a C₁₈ column to perform Edman degradation sequencing on purified peptides. Chromatographic separation allowed the effective purification of only four peptides (Tb-2081, Tb-2671, Tb-3187 and Tb-4018) since there were generally several components per eluting fraction. In parallel, the crude venom was submitted to LC-MS/MS using an orbitrap mass spectrometer for a bottom-up sequencing which yielded 1,778 sequence tags (data not shown). Most of these peptide tags were actually related to a limited number of peptide diversity and corresponded to fragmented and truncated forms of mature peptides.

**Table 1 - Peptide mass fingerprint of Tetramorium bicarinatum venom.**

| List of peptide masses detected by LC-MS using an LCQ Advantage mass spectrometer. Bold indicates peptides with a relative abundance >1% of the whole venom | | | |
|---|---|---|---|
| **Retention time (min)** | **Mass (Da)** | **Relative abondance (%)** | **Tentative Name** |
| **2.27** | **1555.8** | **1.33** | **Tb-1556** |
| 2.88 | 1428.0 | 0.15 | Tb-1428 |
| 7.39 | 2081.1 | 0.81 | Tb-2081 |
| 11.97 | 2785.4 | 0.26 | Tb-2785 |
| 12.04 | 2734.5 | 0.01 | Tb-2734 |
| 12.35 | 2600.0 | 0.25 | Tb-2600 |
| 12.75 | 2670.5 | 0.89 | Tb-2671 |
| **12.82** | **2467.4** | **1.01** | **Tb-2467** |
| 13.28 | 1523.9 | 0.40 | Tb**-**1524 |
| **13.70** | **1934.0** | **2.04** | **Tb-1934** |
| 15.49 | 1064.7 | 0.77 | Tb-1065 |
| 15.91 | 3074.1 | 0.17 | Tb-3074 |
| **16.41** | **3186.6** | **1.32** | **Tb-3187** |
| **18.05** | **1052.6** | **1.81** | **Tb-1053** |
| **18.18** | **1700.9** | **1.63** | **Tb-1701** |
| 18.98 | 1714.9 | 0.98 | Tb-1715 |
| **19.45** | **1295.7** | **1.95** | **Tb-1296** |
| **19.65** | **2423.2** | **1.18** | **Tb-2423** |
| 19.93 | 1460.8 | 0.94 | Tb-1461 |
| **20.40** | **2604.4** | **1.28** | **Tb-2604** |
| 20.55 | 2323.3 | 0.37 | Tb-2323 |
| 20.68 | 2030.0 | 0.33 | Tb-2030 |
| 20.82 | 1619.0 | 0.94 | Tb-1619 |
| 21.09 | 2570.4 | 0.39 | Tb-2570 |
| **21.09** | **2773.5** | **1.32** | **Tb-2774** |
| 21.85 | 2816.3 | 0.30 | Tb-2816 |
| 21.85 | 2649.5 | 0.51 | Tb-2650 |
| **21.85** | **2852.6** | **1.55** | **Tb-2853** |
| 22.13 | 3135.4 | 0.90 | Tb-3135 |
| 22.46 | 1426.8 | 0.22 | Tb-1427 |
| 22.74 | 1795.1 | 0.20 | Tb-1795 |
| 23.90 | 1821.0 | 0.22 | Tb-1821 |
| **24.11** | **4018.1** | **4.99** | **Tb-4018** |
| **26.13** | **939.3** | **1.68** | **Tb-0939** |
| **26.27** | **1571.3** | **11.40** | **Tb-1571** |
| **27.20** | **2212.3** | **13.28** | **Tb-2212** |
| **27.52** | **2008.4** | **1.59** | **Tb-2008** |
| 28.19 | 2004.2 | 0.27 | Tb-2004 |
| **29.09** | **3019.7** | **1.83** | **Tb-3020** |
| **32.41** | **3324.8** | 7.73 | **Tb-3325** |
| **33.71** | **3871.3** | **1.43** | **Tb-3871** |
| **33.91** | **1650.9** | **7.40** | **Tb-1651** |
| **34.31** | **3882.2** | **2.96** | **Tb-3882** |
| 36.16 | 1075.7 | 0.30 | Tb-1076 |
| 37.37 | **2637.6** | **6.23** | **Tb-2638** |
| **38.30** | **1206.7** | **2.13** | **Tb-1207** |
| **39.03** | **1408.7** | **1.03** | **Tb-1409** |
| **40.10** | **2484.5** | **9.36** | **Tb-2485** |

### 2.2 Venom precursors identification, peptide toxins classification and nomenclature

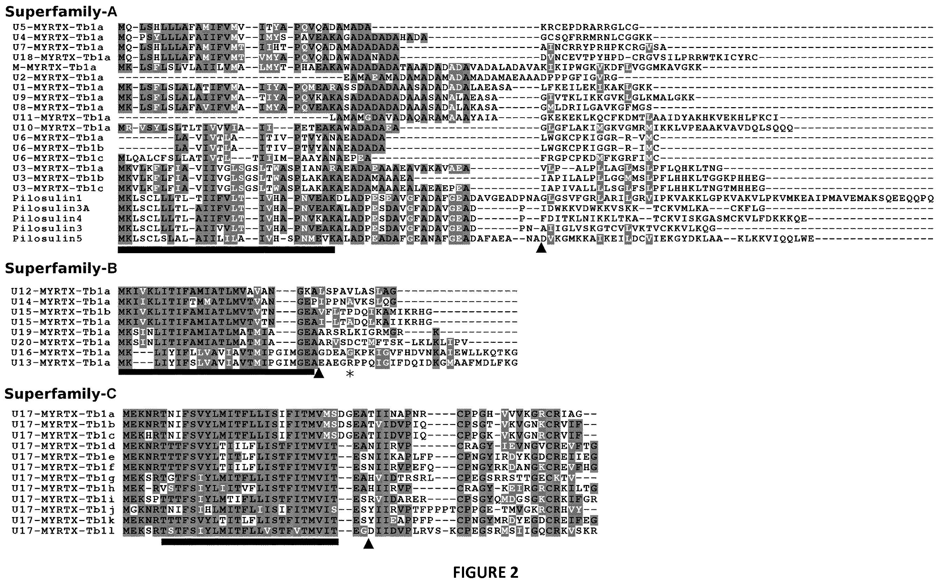

The venomics methods are briefly summarized in the Table S-1. Overall, the venomics approach allowed to assign 43 peptide sequences to the 48 peptide masses initially detected (Table 2). These peptide toxins are encoded by a total number of 29 precursors (including 4 incomplete). Moreover, tblastn of the TSA of *T. bicarinatum* led to eight additional and full-length putative precursors encoding for eight putative mature peptides (Table S-2) increasing the total number of peptides precursors to 37 units (Figure 2).

**Table S-1 - Integrative venomics methodology used to identify the venom peptides of Tetramorium bicarinatum.**

| **Tentative name** | **Mass measured (Da)** | **peaks *de novo* ALC score (%)** | **Edman degradation** | **RT-PCR** | **RNA-Seq** | **Proposed name** | **Mass calculated (Da)** | **deltaMass (Da)** |
|---|---|---|---|---|---|---|---|---|
| Tb-0939 | 939.5785 | 70 | NA | Confirmed | Complete | U₁₂-MYRTX-Tb1a | 939.5753 | 0.0032 |
| Tb-1053 | 1052.5782 | 93 | NA | Confirmed/Incomplete | Incomplete | U₂-MYRTX -Tb1a | 1052.5767 | 0.0015 |
| Tb-1065 | 1064.6355 | 92 | NA | Confirmed | Complete (reassembly) | U₁₄-MYRTX-Tb1a | 1064.6342 | 0.0013 |
| Tb-1076 | 1075.7013 | 92 | NA | Confirmed | Incomplete | U₃-MYRTX-Tb1a | 1075.7005 | 0.0008 |
| Tb-1207 | 1206.7420 | 93 | NA | Confirmed | Incomplete | U₃-MYRTX-Tb1a | 1206.7410 | 0.0010 |
| Tb-1296 | 1295.7369 | 72 | NA | Confirmed | Incomplete | U₃-MYRTX-Tb1a | 1295.7350 | 0.0019 |
| Tb-1409 | 1408.8193 | 80 | NA | Not confirmed | Complete (reassembly) | U₃-MYRTX-Tb1b | 1408.8186 | 0.0007 |
| Tb-1427 | 1426.7787 | 86 | NA | Confirmed | Incomplete | U₃-MYRTX-Tb1a | 1426.7755 | 0.0032 |
| Tb-1428 | - | - | NA | Not confirmed | Not determined | NA | NA | NA |
| Tb-1461 | 1460.7805 | - | NA | Not confirmed | Not determined | NA | NA | NA |
| Tb-1524 | 1523.7247 | - | NA | Confirmed | Complete | U₄-MYRTX-Tb1a | 1523.7020 | 0.0227 |
| Tb-1556 | 1555.7950 | - | NA | JZ168591 * | Incomplete | U₅-MYRTX-Tb1a | 1555.7936 | 0.0014 |
| Tb-1571 | 1571.0204 | 97 | NA | KM030176.1** | Incomplete | U₁-MYRTX-Tb1a | 1571.0174 | 0.0030 |
| Tb-1619 | 1618.0106 | 95 | NA | Confirmed | Complete | U₁₅-MYRTX-Tb1a | 1618.0042 | 0.0064 |
| Tb-1651 | 1650.8586 | 80 | NA | Confirmed / Complete | Incomplete | U₈-MYRTX-Tb1a | 1650.8586 | 0.0000 |
| Tb-1701 | 1700.8827 | - | NA | Not confirmed | Incomplete | U₆-MYRTX-Tb1a | 1700.8789 | 0.0038 |
| Tb-1715 | 1714.8982 | - | NA | Not confirmed | Incomplete | U₆-MYRTX-Tb1b | 1714.8946 | 0.0036 |
| Tb-1795 | 1795.0331 | 91 | NA | Confirmed | Complete | U₁₅-MYRTX-Tb1b | 1795.0291 | 0.0040 |
| Tb-1821 | 1821.0368 | 92 | NA | Confirmed / Complete | Incomplete (reassembly) | U₁₆-MYRTX-Tbac | 1821.0261 | 0.0107 |
| Tb-1934 | 1933.0478 | 84 | NA | Not confirmed | Complete (reassembly) | U₃-MYRTX-Tb1b | 1933.0435 | 0.0043 |
| Tb-2004 | 2003.0369 | 81 | NA | Confirmed | Incomplete | U₁₃-MYRTX-Tb1a | 2003.0299 | 0.0070 |
| Tb-2008 | 2008.3329 | 73 | NA | Confirmed | Complete (reassembly) | U₉-MYRTX-Tb1a | 2008.3322 | 0.0007 |
| Tb-2030 | 2029.9555 | 75 | NA | Not confirmed | Not determined | U₆-MYRTX-Tb1c | 2029.9511 | 0.0044 |
| Tb-2081 | 2081.0667 | - | Confirmed | JZ168710* | Incomplete | U₇-MYRTX-Tb1a | 2081.0636 | 0.0031 |
| Tb-2212 | 2212.3314 | 83 | NA | KF929552.1** | Incomplete | M-MYRTX-Tb1a | 2212.3282 | 0.0032 |
| Tb-2323 | 2323.3010 | 85 | NA | Not confirmed | Complete (reassembly) | U₁₀-MYRTX-Tb1a | 2323.2933 | 0.0077 |
| Tb-2423 | 2422.2590 | 80 | NA | Confirmed/ Completed | Incomplete | U₁₆-MYRTX-Tb1a | 2422.2645 | 0.0055 |
| Tb-2467 | 2467.3585 | - | NA | Confirmed/Complete | Incomplete | U₁₇-MYRTX-Tb1a | 2467.3529 | 0.0056 |
| Tb-2485 | 2484.4676 | 79 | NA | Confirmed/ Complete | Incomplete | U₃-MYRTX-Tb1a | 2484.4657 | 0.0019 |
| Tb-2570 | 2570.3777 | 71 | NA | Confirmed/Corrected | Complete/Ponctual mistakes | U₁₇-MYRTX-Tb1b | 2570.3713 | 0.0064 |
| Tb-2600 | 2600.4473 | - | NA | Not confirmed | Not determined | NA | NA | NA |
| Tb-2604 | 2604.3461 | - | NA | Confirmed/Complete | Incomplete | U₁₇-MYRTX-Tb1d | 2604.3417 | 0.0044 |
| Tb-2638 | 2636.3219 | 82 | NA | Confirmed / Complete | Incomplete | U₁₃-MYRTX-Tb1a | 2636.3607 | 0.0388 |
| Tb-2650 | 2649.4687 | - | NA | Confirmed | Complete | U₁₇-MYRTX-Tb1c | 2649.4611 | 0.0076 |
| Tb-2671 | 2670.4377 | - | Confirmed | Confirmed/Complete | Incomplete | U₁₇-MYRTX-Tb1a | 2670.4323 | 0.0054 |
| Tb-2734 | 2734.5652 | - | NA | Confirmed/Complete | Incomplete | U₁₇-MYRTX-Tb1h | 2734.5588 | 0.0064 |
| Tb-2774 | 2773.4542 | - | NA | Confirmed/Corrected | Complete/Ponctual mistakes | U₁₇-MYRTX-Tb1b | 2773.4507 | 0.0035 |
| Tb-2785 | 2785.3732 | - | NA | Confirmed | Complete | U₁₇-MYRTX-Tb1g | 2785.3712 | 0.0020 |
| Tb-2816 | 2816.4882 | - | NA | Not confirmed | Not determined | NA | NA | NA |
| Tb-2853 | 2852.5460 | - | NA | Confirmed | Complete | U₁₇-MYRTX-Tb1c | 2852.5405 | 0.0055 |
| Tb-3020 | 3019.6734 | - | NA | Confirmed / Complete | Incomplete | U₁₆-MYRTX-Tb1a | 3019.6447 | 0.0287 |
| Tb-3074 | 3072.5415 | - | NA | Confirmed | Complete | U₁₇-MYRTX-Tb1f | 3072.5287 | 0.0128 |
| Tb-3135 | 3134.5739 | - | NA | Confirmed/Corrected | Complete/Ponctual mistakes | U₁₇-MYRTX-Tb1e | 3133.5841 | 0.9898 |
| Tb-3187 | 3186.5773 | - | Confirmed | Confirmed | Complete | U₁₇-MYRTX-Tb1f | 3186.5716 | 0.0057 |
| Tb-3325 | 3323.8513 | 87 | NA | Not confirmed | Complete (reassembly) | U₃-MYRTX-Tb1b | 3323.8515 | 0.0002 |
| Tb-3871 | - | - | NA | Not confirmed | Not determined | NA | NA | NA |
| Tb-3882 | 3882.1811 | 72 | NA | Not confirmed | Complete (reassembly) | U₁₀-MYRTX-Tb1a | 3882.1773 | 0.0038 |
| Tb-4018 | 4018.1465 | - | Confirmed | confirmed/Incomplete | Incomplete | U₁₁-MYRTX-Tb1a | 4018.1359 | 0.0106 |

**Table S-2 - Putative supplementary toxins inferred from transcriptomic analysis.**

| **Proposed toxin name** | **Sequence** | **PTMs** | **Mass calculated (Da)** | **Expression in venom gland (RT-PCR)** |
|---|---|---|---|---|
| U₃-MYRTX- | IAPIVALLLLSGLFSLPFLHHKLTNGTMHHE | NH₂ | 3417.8745 | Not confirmed |
| U₁₇-MYRTX- | RVIDARERCPSGYQMDGSGKCRKIFGR | NH₂ / | 3081.5284 | Confirmed |
| U₁₇-MYRTX- | YIIRVPTFPPPTCPPGETMVGKRCRHVY | 1S-S | 3211.6245 | Not confirmed |
| U₁₇-MYRTX- | YIIEAPPFPCPNGYMRDYEGDCREIFE | NH₂ / | 3220.4043 | Not confirmed |
| U₁₇-MYRTX- | DIIDVPLRVSKCPEGSRMSIIGQCRKVSKR | 1S-S | 3367.8003 | Confirmed |
| U₁₈-MYRTX- | DVNCEVTPYHPDCRGVSILPRRWTKICYRC | 2S-S | 3574.6843 | Not confirmed |
| U₁₉-MYRTX- | ARSRLKIGRM | NH₂ | 1185.7241 | Confirmed |
| U₂₀-MYRTX- | ARVSDCTMFTSKLKLKLIPV | | 2248.2561 | Confirmed |

For all precursors, mature sequences were located at the C-terminal extremity, the alignment of the precursors sequences permitted us to cluster the venom peptide encoding gene transcripts (Figure 2). On the basis of the prepro-peptide sequence identity, the 37 venom peptide precursors were categorized into three different superfamilies (superfamily A-C). Subsequently, examination of the mature region showed that the peptide toxins could be further separated into 21 groups or singletons based on amino acid sequence identity (Table 2 and Table S-2). We assigned 20 groups of peptide sequences with unknown biological activity and pharmacological target that were noted with a "U₁-U₂₀" prefix, while the membrane-active peptide named bicarinalin, was denoted with a "M" prefix. We chose to follow the rational nomenclature specifically developed for naming ant venom peptides that defined Myrmicitoxin (MYRTX) as the reference name suggested for the toxin peptides isolated from the ant subfamily Myrmicinae. Then, the toxin name was followed with the initial of genus and species (Tb) and an alpha-numeric code to distinguish different isotoxins in the same group (i.e. 1a, 1b, 1c, ...).

Finally, we calculated the theoretical molecular weight of each putative mature peptide taking into account the post-translational modifications that were detected by mass spectrometry or deduced from their precursor sequences (Table S-1 and Table S-2). The presence of peptides in the crude venom has been validated by matching the masses observed in mass spectrometry with those predicted from both transcriptomic and proteomic sequencing data. The expression of genes encoding the precursors in the venom glands was also confirmed by RT-PCR for 29 over the 37 precursors (Table S-1).

Below, we describe the Myrmicitoxins identified in the present work, their precursor superfamily membership, their precursor processing, their post-translational modifications, and their homologies with other hymenoptera toxins.

### 2.2.1 Superfamily-A - Pilosulin-like peptide

This superfamily included 17 MYRTX precursors (prepro-M-Tbla, -U₁-Tb1a, -U₂-Tb1a,-U₃-Tb1a, -U₃-Tb1b, -U₃-Tb1c, -U₄-Tb1a, -U₅-Tb1a, -U₆-Tb1a, -U₆-Tb1b, -U₆-Tb1c, -U₇-Tb1a, -U₈-Tb1a, -U₉-Tb1a, -U₁₀-Tb1a, -U₁₁-Tb1a and -U₁₈-Tb1a), including four partially determined precursors (prepro-U₂-Tb1a, -U₆-Tb1a, -U₆-Tb1b and -U₁₁-Tb1a) (Figure 2). The consensus sequence of the prepropeptide has been defined as SEQ ID No. 95 or SEQ ID No. 1). The prepropeptide of these precursors shared about 50% identity with the consensus sequence of the superfamily-A even though the prepro-U₃-Tb1a, -U₃-Tblb, -U₃-Tb1c and -U₆-Tb1c precursors were more dissimilar exhibiting less than 38% of identity with the consensus sequence. Consequently, we have subdivided the superfamily-A into three families as follows; A1 family being composed of prepro-M-Tbla, -U₁-Tb1a, -U₂-Tb1a, -U₄-Tb1a, -U₅-Tb1a, -U₆-Tb1a, -U₆-Tb1b, -U₇-Tb1a, -U₈-Tb1a,-U₉-Tb1a, -U₁₀-Tb1a, -U₁₁-Tb1a, and -U₁₈-Tb1a; A2 family which included prepro-U₃-Tbla, -U₃-Tb1b, and -U₃-Tb1c; and the A3 family represented by the unique full-length precursor prepro-U₆-Tb1c (Table S-3).

This superfamily was closely related to the pilosulin precursors described by Inagaki which shared 35% of sequence identity with the consensus prepro-sequence of superfamily-A and 44% sequence identity with the consensus sequence of A1 family (Figure 2).

Most of the superfamily-A prepropeptide harbored the conserved motif ADADA (SEQ ID No. 10) with an additional C-terminal extension (between 0 to 22 aa) before the mature sequence. The prepropeptide sequences have been submitted to Phobius program to predict transmembrane and signal sequence domain. For all the precursors of superfamily-A, the N-terminal part (from 1 to 24-29 position) was predicted to be a signal sequence while the remaining part that included both pro-region and mature peptide was predicted as a non-cytosolic domain. The cleavage site between the prepropeptide and the mature region was a conserved XAXA (SEQ ID No. 11) excepted for U₃-Tb1c et U₆-Tb1c (Figure 2). Nine MYRTX precursors possessed the GKK motif or G as an extra C-terminal signal for amidation.

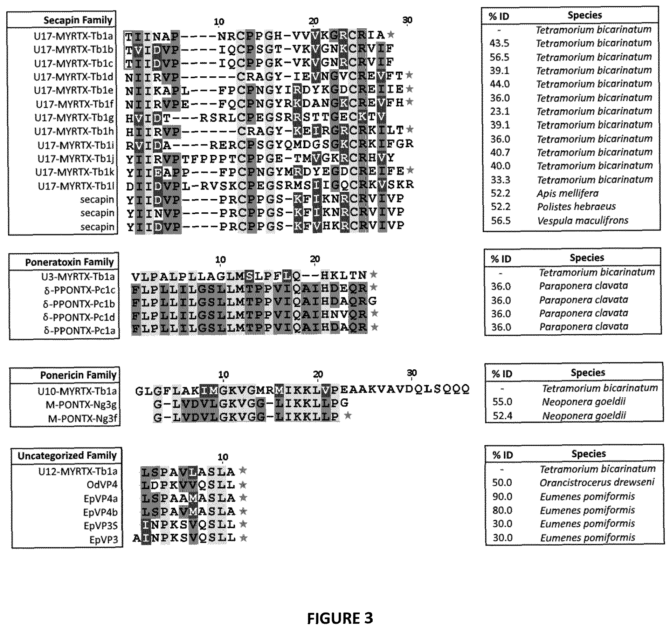

The mature peptides belonging to the superfamily-A of precursors had quite divergent primary sequences with no strictly conserved features in term of amino acid composition, molecular size or cysteine residues (Table 2). Interestingly, two peptides described here had sequence homologies with other ant venom peptides. The peptide U₃-MYRTX-Tb1a possessed 36% of sequence identity with the poneratoxin (δ-PPONTX-Pc1a) a 25-residue linear peptide from *Paraponera clavata* while the peptide U₁₀-MYRTX-Tb1a had 55 and 52% of sequence identity with ponericin G6 and G7 (M-PONTX-Ng3f and M-PONTX-Ng3g) isolated from the venom of *Neoponera goeldii* (Figure 3). Except for U₃-MYRTX-Tb1a and U₁₀-MYRTX-Tb1a, the other peptides described in the

**TABLE 2**

| **Biological group** | **entative name** | **Proposed toxin name** | **Relative abundance** (%) | **Sequence** | **SED No.** | **PTMs** | **Net charge^{a}** | **Hydrophobic aa (%) ^{b}** | **pI^{c}** |
|---|---|---|---|---|---|---|---|---|---|
| M | Tb-2212 | M-MYRTX- | 13.28 | KIKIPWGKVKDFLVGGMKAV | 93 | NH₂ | 5.0 | 50.00 | 10.18 |
| U₁ | Tb-1571 | U₁-MYRTX- | 11.40 | LFKEILEKIKAKL | 94 | NH₂ | 3.0 | 53.85 | 9.53 |
| U₂ | Tb-1053 | U₂-MYRTX- | 1.81 | DPPPGFIGVR | 51 | NH₂ | 1.0 | 30.00 | 5.84 |
| U₃ | Tb-2485 | U₃-MYRTX- | 9.36 | VLPALPLLAGLMSLPFLQHKLTN | 52 | NH₂ | 2.1 | 56.52 | 8.73 |
| | Tb-1207 | **U₃-MYRTX-** | 2.13 | VLPALPLLAGLM | 53 | | 0.0 | 75.00 | 5.49 |
| | Tb-1076 | **U₃-MYRTX-** | 0.30 | VLPALPLLAGL | 54 | | 0.0 | 72.73 | 5.49 |
| | Tb-1428 | **U₃-MYRTX-** | 0.15 | MSLPFLQHKLTN | 55 | NH₂ | 2.1 | 41.67 | 8.52 |
| | Tb-1296 | **U₃-MYRTX-** | 1.95 | SLPFLQHKLTN | 56 | NH₂ | 2.1 | 36.36 | 8.49 |
| | Tb-3325 | U₃-MYRTX- | 7.73 | IAPILALPLLGGMMSLPFLHHKLTGGKPHHE | 57 | NH₂ | 2.4 | 45.16 | 8.62 |
| | Tb-1934 | **U₃-MYRTX-** | 2.04 | SLPFLHHKLTGGKPHHE | 58 | NH₂ | 2.4 | 23.23 | 8.40 |
| | Tb-1409 | **U₃-MYRTX-** | 1.03 | IAPILALPLLGGMM | 59 | | 0.0 | 71.43 | 5.52 |
| U₄ | Tb-1524 | U₄-MYRTX- | 0.40 | GCSQFRRMRNLCG | 60 | NH₂/1S-S | 3.9 | 38.46 | 10.41 |
| U₅ | Tb-1556 | U₅-MYRTX- | 1.33 | KRCEPDRARRGLC | 61 | NH₂/1S-S | 3.9 | 30.77 | 9.69 |
| U₆ | Tb-1701 | U₆-MYRTX- | 1.63 | LWGKCPKIGGRRVMC | 62 | 1S-S | 3.9 | 46.57 | 10.10 |
| | Tb-1715 | U₆-MYRTX- | 0.98 | LWGKCPKIGGRRIMC | 63 | 1S-S | 3.9 | 46.57 | 10.10 |
| | Tb-2030 | U₆-MYRTX- | 0.33 | FRGPCPKDMFKGRFIMC | 64 | 1S-S | 2.9 | 47.06 | 10.03 |
| U₇ | Tb-2081 | U₇-MYRTX- | 0.81 | AINCRRYPRHPKCRGVSA | 65 | 1S-S | 5.0 | 38.89 | 10.86 |
| U₈ | Tb-1651 | U₈-MYRTX- | 7.40 | GMLDRILGAVKGFMGS | 66 | | 1.0 | 50.00 | 8.75 |
| U₉ | Tb-2008 | U₉-MYRTX- | 1.59 | GIVTKLIKKGVKLGLKMAL | 67 | NH₂ | 6.0 | 52.63 | 10.60 |
| U₁₀ | Tb-3882 | U₁₀-MYRTX- | 2.96 | GLGFLAKIMGKVGMRMIKKLVPEAAKVAVDQLSQQQ | 68 | | 4.0 | 50.00 | 10.17 |
| | Tb-2323 | **U₁₀-MYRTX-** | 0.37 | MIKKLVPEAAKVAVDQLSQQQ | 69 | | 1.0 | 47.62 | 8.25 |
| U₁₁ | Tb-4018 | U₁₁-MYRTX- | 4.99 | GKEKEKLKQCFKDMTLAAIDYAKHKVEKHLFKCI | 70 | 1S-S | 4.1 | 44.12 | 9.30 |
| U₁₂ | Tb-0939 | U₁₂-MYRTX- | 1.68 | LSPAVLASLA | 71 | NH₂ | 1.0 | 70.00 | 10.00 |
| U₁₃ | Tb-2638 | U₁₃-MYRTX- | 6.23 | RPPQIGIFDQIDKGMAAFMDLFK | 7273 | NH₂ | 1.0 | 47.83 | 6.04 |
| | Tb-2004 | **U₁₃-MYRTX-** | 0.27 | RPPQIGIFDQIDKGMAAF | 73 | | 0.0 | 44.44 | 5.96 |
| U₁₄ | Tb-1065 | U₁₄-MYRTX- | 0.77 | IPPNAVKSLQ | 74 | NH₂ | 2.0 | 40.00 | 10.00 |
| U₁₅ | Tb-1619 | U₁₅-MYRTX- | 0.94 | ILTADQLKAIIKRH | 75 | NH₂ | 3.1 | 50.00 | 9.99 |
| | Tb-1795 | U₁₅-MYRTX- | 0.20 | VFLTPDQIKAMIKRH | 76 | NH₂ | 3.1 | 46.07 | 9.99 |
| U₁₆ | Tb-3020 | U₁₆-MYRTX- | 1.83 | GDEAGKPKIGVFHDVNKAIEWLLKQTK | 77 | NH₂ | 2.1 | 37.04 | 8.39 |
| | Tb-2423 | **U₁₆-MYRTX-** | 1.18 | GDEAGKPKIGVFHDVNKAIEWL | 78 | | -0.9 | 40.91 | 5.48 |
| | Tb-1821 | **U₁₆-MYRTX-** | 0.22 | HDVNKAIEWLLKQTK | 79 | NH₂ | 2.1 | 40.00 | 8.51 |
| U₁₇ | Tb-2467 | **U₁₇-MYRTX-** | 1.01 | TIINAPNRCPPGHVVVKGRCRIA | 80 | NH₂/1S-S | 5.0 | 43.48 | 10.79 |
| | Tb-2671 | U₁₇-MYRTX- | 0.89 | TIINAPNRCPPGHVVVKGRCRIA | | NH₂ / O- | 5.0 | 43.48 | 10.79 |
| | Tb-2570 | **U₁₇-MYRTX-** | 0.39 | TVIDVPIQCPSGTVKVGNKCRVIF | 81 | 1S-S | 2.9 | 45.83 | 8.88 |
| | Tb-2774 | U₁₇-MYRTX- | 1.32 | TVIDVPIQCPSGTVKVGNKCRVIF | | O-glycosylation / | 2.9 | 45.83 | 8.88 |
| | Tb-2650 | **U₁₇-MYRTX-** | 0.21 | TIIDVPIQCPPGKVKVGNRCRVIF | 82 | 1S-S | 3.9 | 45.83 | 9.50 |
| | Tb-2853 | U₁₇-MYRTX- | 1.55 | TIIDVPIQCPPGKVKVGNRCRVIF | | O-glycosylation / | 3.9 | 45.83 | 9.50 |
| | Tb-2604 | U₁₇-MYRTX- | 1.28 | NIIRVPCRAGYIEVNGVCREVFT | 83 | NH₂/1 S-S | 1.9 | 52.17 | 8.06 |
| | Tb-3135 | U₁₇-MYRTX- | 0.90 | NIIKAPLFPCPNGYIRDYKGDCREIIE | 84 | 1S-S | 0.9 | 44.44 | 6.20 |
| | Tb-3187 | U₁₇-MYRTX- | 1.32 | NIIRVPEFQCPNGYRKDANGKCREVFH | 85 | NH₂/1S-S | 3.0 | 37.04 | 8.86 |
| | Tb-3074 | **U₁₇-MYRTX-** | 0.17 | IIRVPEFQCPNGYRKDANGKCREVFH | 86 | NH₂/1S-S | 3.0 | 38.46 | 8.86 |
| | Tb-2785 | U₁₇-MYRTX- | 0.26 | HVIDTRSRLCPEGSRRSTTGECKTV | 87 | 1S-S | 2.0 | 24.00 | 8.96 |
| | Tb-2734 | U₁₇-MYRTX- | 0.01 | HIIRVPCRAGYKEIRGRCRKILT | 88 | NH₂/1S-S | 7.0 | 43.48 | 10.90 |

| | | | | | | | | | |
|---|---|---|---|---|---|---|---|---|---|
| ^{a}according to PepCalc (https://pepcalc.com/), ^{b}acconding to expasy (https://web.expasy.org/compute pi/), ^{c}according to | | | | | | | | | |

superfamily-A of precursors had no similarities with previously described ant venom peptides. Interestingly, despite the superfamily-A was assigned to pilosulin precursors, its mature peptides were very divergent. Sequence analysis of mature peptides from the superfamily-A also revealed that several short peptides were fragments of larger peptides and belonged to the same toxin precursor. The U₃-MYRTX group of peptides is produced by three homologous peptide precursors that yield the full-length mature peptides (U₃-Tb1a, U₃-Tb1b and U₃-Tb1c) (Table 2 and Table S-3), plus additional truncated versions of these peptides (Tb-1207, Tb-1076, Tb-1428, Tb-1296, Tb-1934 and Tb-1409) (Table 2). Similarly to U₃-MYRTXs, we also discovered that TB2323 was a fragment of the peptide U₁₀-Tb1a (Table 2). Therefore, the maturation of 17 precursors of the superfamily-A generated 22 peptides that were confirmed by mass spectrometry as present in the venom (Table 2). An additional putative mature peptide with two disulfide bonds was predicted to be generated by the U₁₈-MYRTX-Tb1a precursor even though its presence in the crude venom was not confirmed by mass spectrometry (Table S-2).

**Table S-3 - Molecular features of the toxin precursors expressed in the venom glands of the ant Tetramorium bicarinatum.**

| **Precursor superfamily** | **Family** | **Toxin precursor** | **Precursor length** | **Mature peptide length** | **Prepropeptide motif signal** | **C-ter amidation motif** |
|---|---|---|---|---|---|---|
| | A1 | Prepro-M-MYRTX-Tb1a | 79 aa | 20 aa | DAVA | GKK |
| | A1 | Prepro-U₁-MYRTX-Tb1a | 68 aa | 13 aa | EASA | GKK |
| | A1 | Prepro-U₂-MYRTX-Tb1a | Incomplete | 10 aa | EAAA | G |
| | A1 | Prepro-U₄-MYRTX-Tb1a | 56 aa | 13 aa | HADA | GKK |
| | A1 | Prepro-U₅-MYRTX-Tb1a | 45 aa | 13 aa | MADA | G |
| | A1 | Prepro-U₆-MYRTX-Tb1a | Incomplete | 15 aa | DADA | |
| | A1 | Prepro-U₆-MYRTX-Tb1b | Incomplete | 15 aa | | |
| | A1 | Prepro-U₇-MYRTX-Tb1a | 54 aa | 18 aa | DADA | |
| **A** | A1 | Prepro-U₈-MYRTX-Tb1a | 66 aa | 16 aa | KASA | |
| | A1 | Prepro-U₉-MYRTX-Tb1a | 72 aa | 19 aa | EASA | GKK |
| | A1 | Prepro-U₁₀-MYRTX-Tb1a | 70 aa | 36 aa | ADAE | |
| | A1 | Prepro-U₁₁-MYRTX-Tb1a | Incomplete | 34 aa | YAIA | |
| | A1 | Prepro-U₁₈-MYRTX-Tb1a | 65 aa | 30 aa | NANA | |
| | A2 | Prepro-U₃-MYRTX-Tb1a | 73 aa | 23 aa | VAEA | G |
| | A2 | Prepro-U₃-MYRTX-Tb1b | 73 aa | 31 aa | AAEA | G |
| | A2 | Prepro-U₃-MYRTX-Tb1c | 81 aa | 31 aa | EPEA | G |
| | A3 | Prepro-U₆-MYRTX-Tb1c | 50 aa | 17 aa | EPEA | |
| **B** | B1 | Prepro-U₁₂-MYRTX-Tb1a | 36 aa | 10 aa | NGKA | G |
| | B1 | Prepro-U₁₄-MYRTX-Tb1a | 36 aa | 10 aa | NGEP | G |
| | B1 | Prepro-U₁₅-MYRTX-Tb1a | 41 aa | 14 aa | NGEA | G |
| | B1 | Prepro-U₁₅-MYRTX-Tb1b | 41 aa | 15 aa | NGEA | G |
| | B1 | Prepro-U₁₉-MYRTX-Tb1a | 38 aa | 10 aa | AGEA | GRK |
| | B1 | Prepro-U₂₀-MYRTX-Tb1a | 45 aa | 20 aa | AGEA | |
| | B2 | Prepro-U₁₆-MYRTX-Tb1a | 55 aa | 27 aa | MGEA | G |
| | B2 | Prepro-U₁₃-MYRTX-Tb1a | 55 aa | 23 aa | EAEG | G |
| **C** | | Prepro-U₁₇-MYRTX-Tb1a | 57 aa | 23 aa | DGEA | G |
| | | Prepro-U₁₇-MYRTX-Tb1b | 58 aa | 24 aa | DSEA | |
| | | Prepro-U₁₇-MYRTX-Tb1c | 58 aa | 24 aa | DGEA | |
| | | Prepro-U₁₇-MYRTX-Tb1d | 55 aa | 23 aa | ITEA | G |
| | | Prepro-U₁₇-MYRTX-Tb1e | 59 aa | 27 aa | ITES | G |
| | | Prepro-U₁₇-MYRTX-Tb1f | 59 aa | 27 aa | ITES | G |
| | | Prepro-U₁₇-MYRTX-Tb1g | 56 aa | 25 aa | ITEA | |
| | | Prepro-U₁₇-MYRTX-Tb1h | 54 aa | 23 aa | ITEA | G |
| | | Prepro-U₁₇-MYRTX-Tb1i | 57 aa | 27 aa | ITES | G |
| | | Prepro-U₁₇-MYRTX-Tb1j | 59 aa | 28 aa | ISES | |
| | | Prepro-U₁₇-MYRTX-Tb1k | 59 aa | 27 aa | ITES | G |
| | | Prepro-U₁₇-MYRTX-Tb1l | 60 aa | 30 aa | ITEG | |

| | | | | | | |
|---|---|---|---|---|---|---|
| Aa = Amino acid | | | | | | |

### 2.2.2 Superfamily-B - New ant venom precursors

The superfamily-B which included eight precursors (Figure 2) was subdivided into B1 (prepro-U₁₂-Tbla, -U₁₄-Tb1a, -U₁₅-Tb1a, -U₁₅-Tb1b, -U₁₉-Tb1a and -U₂₀-Tb1a) and B2 (prepro-U₁₆-Tb1a and - U₁₃-Tb1a) families (Table S-3). The precursors had a consensus prepro-sequence defined as SEQ ID No. 96 and more preferably as SEQ ID No. 2 and shared about 63% of identity with this consensus sequence. The consensus sequence showed 13% of identity with the consensus prepropeptide sequence of the superfamily-A and had no significant homology to any previously known ant venom peptide sequences. Consequently, the superfamily-B should be considered as a new ant venom precursor superfamily.

The expression of prepro-U₁₉-Tb1a and -U₂₀-Tb1a in the venom glands was confirmed by RT-PCR even though the theoretical masses of their mature peptides have not been detected in the crude venom by mass spectrometry. The first 25 amino acids at the N-terminus of these precursors were predicted as a signal sequence by the Phobius program while the remaining C-terminal part corresponding to mature peptide was predicted as a non-cytosolic domain. The mature peptides were cleaved after a XGEA (SEQ ID No. 12), NGEP (SEQ ID No. 13), EAEG (SEQ ID No. 14) or NGKA ((SEQ ID No. 15) motifs to produce peptides ranging from 10 to 27 amino acids (Figure 2). Additional cleavages of the two mature peptides U₁₃-Tb1a and U₁₆-Tb1a resulted in their corresponding short versions Tb-2004, and Tb-2423 and Tb-1821, respectively (Table 2).

There was no significant similarity between the precursors from superfamily-B and other venom precursors. However, despite a very distinct prepropeptide region (only 28% of identity), the mature peptide U₁₂-MYRTX-Tb1a and the peptide VP4a, isolated from the solitary wasp *Eumenes pomiformis* (UniProt accession number: D1MEJO), are very similar with 90% of sequence identity (Figure 3).

**Table 2 - Myrmicitoxin sequences from the venom of Tetramorium bicarinatum venom**

| **Biological group** | **Tentative name** | **Proposed toxin name** | **Relative abundance (%)** | **Sequence** | **PTMs** | **Net charge^{a}** | **Hydrophobic aa (%) b** | **pI^{c}** |
|---|---|---|---|---|---|---|---|---|
| M | Tb-2212 | M- | 13.28 | KIKIPWGKVKDFLVGGMKAV | NH₂ | 5.0 | 50.00 | 10.1 |
| U₁ | Tb-1571 | U₁- | 11.40 | LFKEILEKIKAKL | NH₂ | 3.0 | 53.85 | 9.53 |
| U₂ | Tb-1053 | U₂- | 1.81 | DPPPGFIGVR | NH₂ | 1.0 | 30.00 | 5.84 |
| U₃ | Tb-2485 | U₃- | 9.36 | VLPALPLLAGLMSLPFLQHKLTN | NH₂ | 2.1 | 56.52 | 8.73 |
| | Tb-1207 | **U₃-** | 2.13 | VLPALPLLAGLM | | 0.0 | 75.00 | 5.49 |
| | Tb-1076 | **U3-** | 0.30 | VLPALPLLAGL | | 0.0 | 72.73 | 5.49 |
| | Tb-1428 | **U3-** | 0.15 | MSLPFLQHKLTN | NH₂ | 2.1 | 41.67 | 8.52 |
| | Tb-1296 | **U3-** | 1.95 | SLPFLQHKLTN | NH₂ | 2.1 | 36.36 | 8.49 |
| | Tb-3325 | U₃- | 7.73 | IAPILALPLLGGMMSLPFLHHKLTGGKPHH | NH₂ | 2.4 | 45.16 | 8.62 |
| | Tb-1934 | **U₃-** | 2.04 | SLPFLHHKLTGGKPHHE | NH₂ | 2.4 | 23.23 | 8.40 |
| | Tb-1409 | **U3-** | 1.03 | IAPILALPLLGGMM | | 0.0 | 71.43 | 5.52 |
| U₄ | Tb-1524 | U₄- | 0.40 | GCSQFRRMRNLCG | NH₂/1S-S | 3.9 | 38.46 | 10.4 |
| U₅ | Tb-1556 | U₅- | 1.33 | KRCEPDRARRGLC | NH₂/1S-S | 3.9 | 30.77 | 9.69 |
| U₆ | Tb-1701 | U₆- | 1.63 | LWGKCPKIGGRRVMC | 1S-S | 3.9 | 46.57 | 10.1 |
| | Tb-1715 | U₆- | 0.98 | LWGKCPKIGGRRIMC | 1S-S | 3.9 | 46.57 | 10.1 |
| | Tb-2030 | U₆- | 0.33 | FRGPCPKDMFKGRFIMC | 1S-S | 2.9 | 47.06 | 10.0 |
| U₇ | Tb-2081 | U₇- | 0.81 | AINCRRYPRHPKCRGVSA | 1S-S | 5.0 | 38.89 | 10.8 |
| U₈ | Tb-1651 | U₈- | 7.40 | GMLDRILGAVKGFMGS | | 1.0 | 50.00 | 8.75 |
| U₉ | Tb-2008 | U₉- | 1.59 | GIVTKLIKKGVKLGLKMAL | NH₂ | 6.0 | 52.63 | 10.6 |
| U₁₀ | Tb-3882 | U₁₀- | 2.96 | GLGFLAKIMGKVGMRMIKKLVPEAAKVA | | 4.0 | 50.00 | 10.1 |
| | Tb-2323 | **U₁₀-** | 0.37 | MIKKLVPEAAKVAVDQLSQQQ | | 1.0 | 47.62 | 8.25 |
| U₁₁ | Tb-4018 | U₁₁- | 4.99 | GKEKEKLKQCFKDMTLAAIDYAKHKVEK | 1S-S | 4.1 | 44.12 | 9.30 |
| U₁₂ | Tb-0939 | U₁₂- | 1.68 | LSPAVLASLA | NH₂ | 1.0 | 70.00 | 10.0 |
| U₁₃ | Tb-2638 | U₁₃- | 6.23 | RPPQIGIFDQIDKGMAAFMDLFK | NH₂ | 1.0 | 47.83 | 6.04 |
| | Tb-2004 | **U₁₃-** | 0.27 | RPPQIGIFDQIDKGMAAF | | 0.0 | 44.44 | 5.96 |
| U₁₄ | Tb-1065 | U₁₄- | 0.77 | IPPNAVKSLQ | NH₂ | 2.0 | 40.00 | 10.0 |
| U₁₅ | Tb-1619 | U₁₅- | 0.94 | ILTADQLKAIIKRH | NH₂ | 3.1 | 50.00 | 9.99 |
| | Tb-1795 | U₁₅- | 0.20 | VFLTPDQIKAMIKRH | NH₂ | 3.1 | 46.07 | 9.99 |
| U₁₆ | Tb-3020 | U₁₆- | 1.83 | GDEAGKPKIGVFHDVNKAIEWLLKQTK | NH₂ | 2.1 | 37.04 | 8.39 |
| | Tb-2423 | **U₁₆-** | 1.18 | GDEAGKPKIGVFHDVNKAIEWL | | -0.9 | 40.91 | 5.48 |
| | Tb-1821 | **U₁₆-** | 0.22 | HDVNKAIEWLLKQTK | NH₂ | 2.1 | 40.00 | 8.51 |
| U₁₇ | Tb-2467 | **U₁₇-** | 1.01 | TIINAPNRCPPGHVVVKGRCRIA | NH₂/1S-S | 5.0 | 43.48 | 10.7 |
| | Tb-2671 | U₁₇- | 0.89 | TIINAPNRCPPGHVVVKGRCRIA | NH₂ / O- | 5.0 | 43.48 | 10.7 |
| | Tb-2570 | **U₁₇-** | 0.39 | TVIDVPIQCPSGTVKVGNKCRVIF | 1S-S | 2.9 | 45.83 | 8.88 |
| | Tb-2774 | U₁₇- | 1.32 | TVIDVPIQCPSGTVKVGNKCRVIF | O- | 2.9 | 45.83 | 8.88 |
| | Tb-2650 | **U₁₇-** | 0.21 | TIIDVPIQCPPGKVKVGNRCRVIF | 1S-S | 3.9 | 45.83 | 9.50 |
| | Tb-2853 | U₁₇- | 1.55 | TIIDVPIQCPPGKVKVGNRCRVIF | O- | 3.9 | 45.83 | 9.50 |
| | Tb-2604 | U₁₇- | 1.28 | NIIRVPCRAGYIEVNGVCREVFT | NH₂/ 1 S-S | 1.9 | 52.17 | 8.06 |
| | Tb-3135 | U₁₇- | 0.90 | NIIKAPLFPCPNGYIRDYKGDCREIIE | 1S-S | 0.9 | 44.44 | 6.20 |
| | Tb-3187 | U₁₇- | 1.32 | NIIRVPEFQCPNGYRKDANGKCREVFH | NH₂/ 1S-S | 3.0 | 37.04 | 8.86 |
| | Tb-3074 | **U₁₇-** | 0.17 | IIRVPEFQCPNGYRKDANGKCREVFH | NH₂/ 1S-S | 3.0 | 38.46 | 8.86 |
| | Tb-2785 | U₁₇- | 0.26 | HVIDTRSRLCPEGSRRSTTGECKTV | 1S-S | 2.0 | 24.00 | 8.96 |
| | Tb-2734 | U₁₇- | 0.01 | HIIRVPCRAGYKEIRGRCRKILT | NH₂/ 1S-S | 7.0 | 43.48 | 10.9 |

| | | | | | | | | |
|---|---|---|---|---|---|---|---|---|
| ^{a}according to PepCalc (https://pepcalc.com/), ^{b}acconding to expasy (https://web.expasy.org/compute pi/), ^{c}according to | | | | | | | | |

### 2.2.3 Superfamily-C - Secapin-like peptides

The 12 precursors of the superfamily-C (Figure 2) yielded MYRTX that shared significant sequence similarity with the secapins, a group of multifunctional peptides found in the venom of numerous hymenoptera (Figure 3). However, the prepropeptide region of these precursors showed no clear sequence similarity with other insect secapin precursors (12% of identity on average between consensus prepropeptide sequence and other insect secapin prepropeptide sequences). Both prepropeptide region and mature sequence of superfamily-C precursors were highly conserved (Figure 2). The consensus prepropeptide sequence was defined SEQ ID No. 3 or SEQ ID No.4 and the consensus cleavage site as GEA, SEA, TEA, TEF, TEG or SEQ ID No. 5to SEQ ID No. 8. The percentage of sequence identity was about 75% between prepropeptide sequences and consensus sequence. The presence in the crude venom of the four mature peptides U₁₇-Tb1i, U₁₇-Tblj, U₁₇-Tb1k and U₁₇-Tb1l was not confirmed by proteomic analysis (Table S-2). The 12 mature peptides were ranging from 23 to 30 amino acids and all contained one disulfide bond. Six peptides were amidated at the C-terminus with an extra glycine residue in the precursor. An alignment of the sequence of the 12 secapin-like peptides from *T. bicarinatum* revealed several conserved features including the two cysteine residues, a pair of isoleucine at the N-terminus, a proline-rich region around the first cysteine immediately followed by a strictly conserved glycine residue, and an arginine/lysine-rich region around the second cysteine (Figure 3). These peptides are clearly paralogs and the consensus sequence of the mature peptides is defined as (SEQ ID No. 9).

Interestingly, the three peptides U₁₇-Tb1a, U₁₇-Tb1b and U₁₇-Tb1c exhibited a primary structure with a 203 Da mass unit lack in comparison to the experimental measurement (Table 2). This mass default was consistent with a O-glycosylation by an N-acetyl-hexosamine (HexNAc). LC-MS analysis of the tryptic fragments of these three peptides revealed that the difference of mass arose from the N-terminal tryptic fragment and more precisely from the common glycosylation site constituted by the N-terminal threonine. Their non-glycosylated counterparts were also present in the venom as Tb-2467, Tb-2570 and Tb-2650 peptides, respectively.

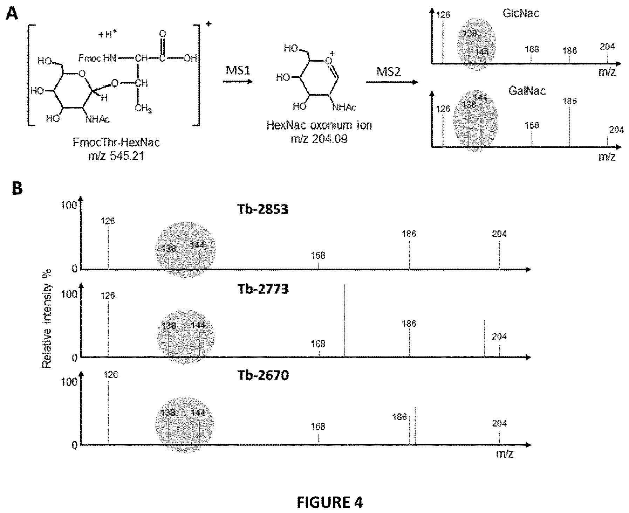

Since O-glycosylation of peptide toxins has been seldom found in animal venoms, we have decided to further investigate of type of *O*-linked HexNAc in U₁₇-Tb1a, U₁₇-Tb1b and U₁₇-Tb1c by assessing the MS-MS fragmentation of their oxonium ions. Indeed, the MS-MS spectrum yields specific fragments that allows the identification of the isomeric forms of sugars. In particular, the ions at 138 and 144 m/z are relevant to disciminate a N-acetylglucosamine (GluNAc) from a N-acetylgalactosamine (GalNAc) since the intensity ratio of these ions significantly differs for each sugar (Figure 4a). After the experimental validation of this approach from models of N-protected GalNAc- and GluNAc-threonine, application to the three glycosylated peptides has unambiguously shown a characteristic ratio in favor to glycosylations by a GalNAc (Figure 4b).

### 3. DISCUSSION

### 3.1 Strategy for the identification of venom peptide from T. bicarinatum

Herein, we describe a large-scale identification of the peptide toxins from the venom of T. *bicarinatum* as well as their expression in the venom glands (Table S-1). As mentioned above, our integrative strategy includes three complementary approaches: a proteomic analysis (Edman degradation and/or mass spectrometry-based sequencing), a direct transcriptomic analysis of the venom glands (RT-PCR and DNA sequencing) and a data mining of the available RNA-seq transcriptome. This venomics approach led to the identification of 37 venom peptide precursors which were classified into three superfamilies (-A, -B and -C). A total number of 29 mature peptides were confirmed in the venom of *T. bicarinatum* by mass spectrometry.

### 3.2 Phylogenetic relationships of venom peptide precursors

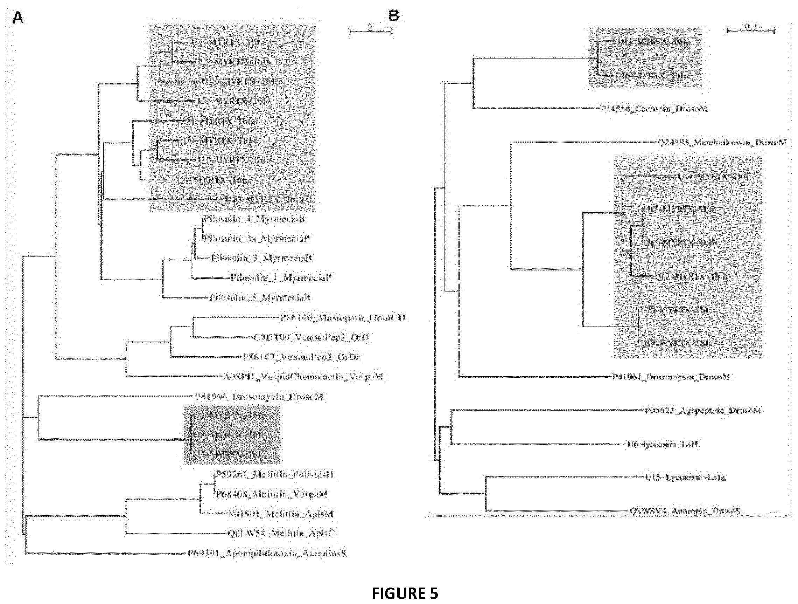

The precursors belonging to the superfamily-A exhibited different lengths due to the number of AD or AE motif occurrences in their prepropeptides regions. The repetition of these motifs is also observed in the pilosulin-related precursors, as well as in the precursors of melittin and α-pompilidotoxin. The melittin precursor shares 35% identity with the U₃-MYRTX precursors (prepro-U₃-Tb1a, -b and -c) while the precursor of α-pompilidotoxin has 30% of sequence identity with U₄-Tb1a precursor. Noteworthy, prepro-U₄-Tb1a was also related to the drosomycin precursor (46% of sequence identity), an antimicrobial peptide found in the hemolymph of *Drosophila melanogaster.* A phylogenetic tree reconstruction from prepropeptide sequences alignment with minimum evolution (ME) statistical method placed prepro-U₃ sequences in the same cluster as drosomycin and α-pompilidotoxin (Figure 5A). As expected other prepropeptide sequences of superfamily-A precursors fell into the same cluster as pilosulin.

Although prepropeptide peptides of the superfamily-B had no obvious similarity with other known venom toxin precursors, the consensus prepropeptide sequence exhibited about 20% identity with prepropeptide sequences of the antimicrobial *Drosophila* peptides cecropin, adropin, drosomycin and metchnikowin, the maximum identity being observed between cecropin and both U₁₆-Tb1a and U₁₃-Tb1a precursors (40% of sequence identity) (Figure 5B). On ME phylogenetic tree, prepro-U₁₃-Tb1a and prepro-U₁₆-Tb1a (precursor family B2) fell into the same cluster as cecropin and precursors from B1 family cluster with metchnikowin (Figure 5B). The superfamily-C precursors maturation generated native peptides related to secapins, a group of multifunctional peptides found in the venom of several hymenoptera and even in the hemolymph of the honey bee as a part of its innate immunity. ADNc encoding secapins have also been characterized in non-venomous insects such as drosophila (dromepin-3, cDNA accession number JX977165) and mosquito (secapin-3, accession number HQ170505, secapin-2a -2b, -2c accession number HQ170502, HQ170503 and HQ170504, respectively). The homologies of *T*. *bicarinatum* venom peptides with other hymenoptera toxins, and more importantly with antimicrobial peptides from the hemolymph of insects, support the hypothesis that hymenoptera venom peptides may be related to their innate immune system.

### 3.3 Evolution of T. bicarinatum venom precursors superfamilies

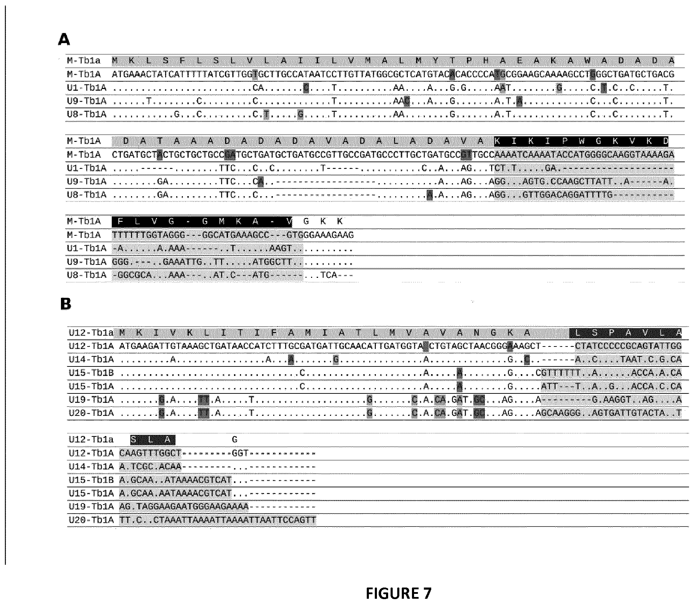

The conserved prepropeptide regions and the variability of mature sequences observed in the superfamilies of *T. bicarinatum* venom precursors suggest that venom peptides have evolved from a relatively small set of ancestral genes presumably through duplication followed by the divergence of copies in the regions encoding mature peptides. For instance, the comparison of nucleotide sequences encoding for M, U₁, U₉, U₈-MYRTX-Tb1a precursors showed a weak number of non-synonymous substitutions (3 to 10) and codon deletion (5 to 6) in the prepro-regions while the sequences of mature regions displayed a high rate of mutation (Figure 7). The comparison of encoding sequences for superfamily-B precursors led to the same observation with 1 to 11 non synonymous substitutions on 85 nucleotides in the prepro-region and an excess of non-synonymous substitutions in their mature sequences (Figure 7).

This conservation of prepropeptide region and divergence of mature region have been observed for frog antimicrobial peptides as well as for venom peptides from spiders and cone snails. A high rate of polymorphisms (intra and interspecific) has also been observed in AMP (Antimicrobial peptide) genes from *Drosophila* genus. Authors hypothesize that this polymorphism within species is linked to selective forces such as infection pathogen pressure. Ant use venoms to capture a broad array of arthropods and also to protect themselves against microbial infection. The divergence in the primary sequence of mature peptides may indicate a range of molecular targets and/or mode of action. Thus, we hypothesized that differential exposure to prey and microbe pathogens has imposed selective pressures toward toxins evolution leading to a high rate of mutations in the mature sequences.

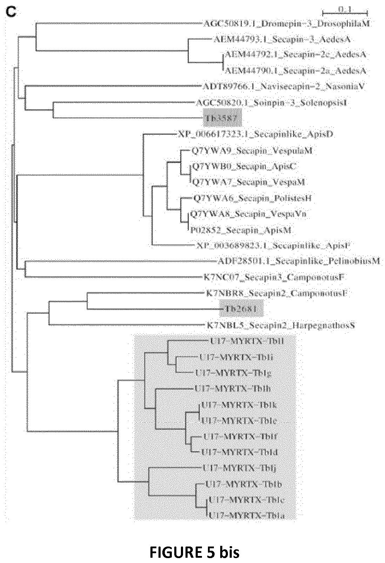

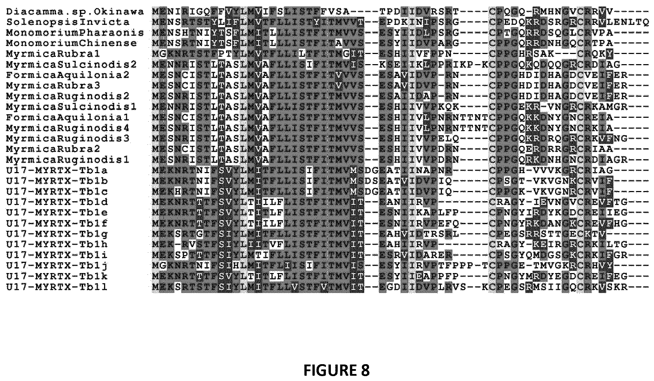

The superfamily-C precursors were related to secapins. Since the description of the first secapin in honeybee venom, several other secapin peptides have been reported in bees, ants, wasps, drosophila, mosquitoes and spiders. Surprisingly, the prepropeptide sequences of *T. bicarinatum* venom secapins have a weak identity with other secapin precursors including secapins described from other ant species. On ME phylogenetic tree generated from prepropeptide sequences of all characterized arthropods secapin those of *T. bicarinatum* venom cluster separately (Figure 5C). However, blast of secapin mature sequences on the transcriptome of *T. bicarinatum* allowed us to predict two other secapin precursors called prepro-Tb3587 and prepro-Tb2681 (contigs GASM01032508.1 and GASM01033385.1). Their prepropeptide sequences cluster on ME phylogenetic tree with wasp navisecapin (ADT89766.1, *Nasonia vitripennis*) and ant soinpin-3 (AGC50820.1, *Solenopsis invicta)*. Moreover, the blast of the prepro-sequences of the *T. bicarinatum* superfamily-C on the transcriptome assemblies available in the NCBI database has led to the identification of multiple novel secapin precursors in *Formicidae.* Thus, 16 novel secapin precursors were found from both *Myrmicinae* and *Formicidae* subfamilies and share about 53% of identity with the secapin consensus prepropeptide sequences of *T. bicarinatum* (Figure 8).

In contrast, the mature region of secapin precursors are well conserved in insects and they share about 34% of sequence identity with the consensus sequence of *T. bicarinatum* mature secapins. This evolutive pressure on mature sequence but not on signal and pro-regions is often observed. For instance, this has been reported in vertebrates for the RFamide 26RFa/QRFP neuropeptide whose the C-terminal extremity that exerts some biological activities is particularly well conserved. The conservation of the mature primary sequences **PPTCP**XG (SEQ ID No. 89) and GX**CR (**(SEQ ID No. 90) may highlight their functional importance in secapin folding and/or target recognition. The diversity of prepropeptide regions in addition to the conservation of mature sequences of secapins lead to hypothesize that convergence mechanisms drive evolution of the genes encoding the different clusters of secapin precursors.

### 3.3 Predicted maturation pathway

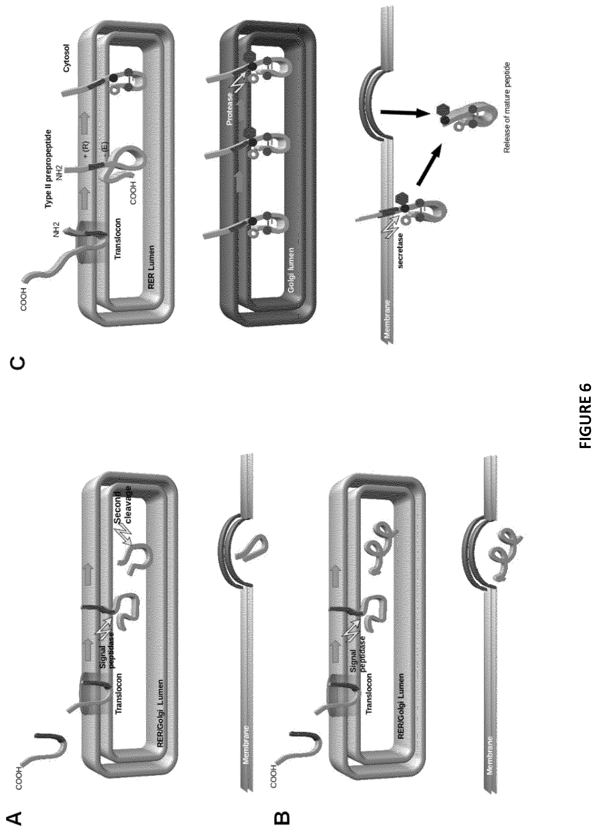

Figure 6 outlines the maturation pathways of the venom peptide precursors that were predicted from the Phobius analysis. For the superfamily-A precursors, the N-terminal signal sequence allows the translocation into the endoplasmic reticulum (ER), after cleavage by a signal peptidase the pro- and the mature regions are released into the lumen. Then, an additional cleavage may occur in the ER or Golgi apparatus to release the mature peptide. This maturation pathway has also been predicted for the pilosulin family, drosomycin, melittin and pompilidotoxin as described on Uniprot database. The predicted maturation pathway of the superfamily-B of precursors is basically the same that of observed for the superfamily-A in which the N-terminal signal sequence is cleaved after translocation in ER, directly releasing the mature peptide in the lumen. This maturation pathway was previously reported for the cecropin and metchnikowin precursors in Uniprot database. Sequence identities described above for the superfamilies-A and -B with other insect peptide precursors are consistent with a conservation of the maturation pathway. For the superfamily-C of precursors, the maturation pathway is markedly different. The N-terminal part of the precursor, predicted as a cytosolic domain, is followed by a transmembrane region acting as an internal signal sequence allowing the C-terminal part of the precursor to be translocated into the ER lumen. Finally, this luminal region undergoes several post-translational modifications such as a O-glycosylation of threonine residues by a GalNAc before releasing the mature peptide. Interestingly, secapin precursors identified in other insect groups are predicted to follow a different maturation pathway as *Tetramorium* secapin precursors that is however identical to that described for *T. bicarinatum* superfamily-A and -B precursors, suggesting that different maturation pathways can produce a same family of mature peptides.

### 3.4 Presumed biological functions of mature peptides

The superfamilies -A and -B of precursors encoded for a set of peptides that displayed a remarkable low sequence identity. Despite this variety, several peptides shared physicochemical properties characteristic of linear AMPs such as amphipathy, polycationicity and α-helix propensity and most are likely membrane-active peptides. The physicochemical properties of AMPs which do not require a strictly conserved pattern of amino acids to be functional may explain the high diversity of toxin sequences observed in the venom peptidome of *T*. *bicarinatum.* Hymenoptera venoms are known to be rich in linear AMPs. For instance, M-poneritoxins-Ng (ponericins), M-poneritoxins-Dq (dinoponeratoxins), M-myrmeciitoxin-Mpla (pilosulin 1), M-ectatotoxin-Eb2a-c (ponericin-Q42, -Q49 and -Q50) and M-MYRTX-Tbla (bicarinalin), which is the most abundant peptide of the *T. bicarinatum* venom, have been characterized as ant AMPs. Furthermore, some peptides described in the present manuscript (U₁₀-Tb1a and U₁₂-Tb1a) exhibited homologies with the antimicrobial and insecticidal poneritoxins-Ng (ponericins) and antimicrobial solitary wasp peptides (VP4a, VP4b) . Altogether, our data suggest that most of the peptides yielded by the superfamilies -A and -B were polycationic and amphiphilic peptides with likely antimicrobial, cytotoxic and insecticidal properties.

The mature peptides belonging to the superfamily-A of precursors were predominant in the *T*. *bicarinatum* venom with more than 74% of the peptidome content and thus, highlighted important functional roles for these myrmicitoxins. Among the superfamily-A, the peptides from the eight U₃-myrmicitoxins represented around 25% of the whole peptidome. The three full-length peptides U₃-MYRTX-Tb1a, -b and -c were markedly different from others peptides of the peptidome in being globally hydrophobic with a positively charged C-terminal extremity (Table 2 and Table S-2). They displayed sequence homologies with the peptides δ-PPONTX-Pc1a (poneratoxin) from the venom of the ant *Paraponera clavata* (Figure 3). δ-PPONTX-Pc1a is a neurotoxic peptide affecting the sodium channels in both vertebrates and invertebrates⁴⁶. These data suggest that the U₃-MYRTX group of peptides may have an important role in the paralysis of prey probably by affecting sodium channels.

The secapin peptides from *Hymenoptera* are known to display several activities including neurotoxic, hyperalgesic, edematogenic, anti-fibrinolytic, anti-elastolytic and anti-bacterial properties. Secapin-like peptides from the superfamily-C of precursors might have similar functions. Interestingly, some secapin-like peptides from the venom of *T. bicarinatum* exhibited O-glycosylation of the N-terminal threonine residue. To the best of our knowledge, such PTM has not been previously reported for ant venom peptides neither for other secapin peptides. Nevertheless, O-glycosylated AMPs, named formaecins, have been isolated in the hemolymph of the ant *Myrmecia gulosa* and were significantly more active than their non-glycosylated counterparts. Other O-glycosylated peptides have also been identified in the venom of cone snails, scorpions, snakes, and wasps. All these glycosylated peptides do not exhibit any significant sequence homology with the glycosylated Myrmicitoxins described in the present study.

### 3.5 Peptides fragmentation and degradation in ant venom

Recent studies using high sensitive mass spectrometry reveal that ant venoms can contain several thousands of peptides. In this study, we have detected more than 2,800 peptide masses in the venom of *T. bicarinatum.* However, each signal does not reflect the presence of an individual peptide in the venom. Indeed, several signals can be assigned to the same peptide like sodium and potassium adducts or correspond to artefactual species such as intermediate fragments or peptides without certain post translational modifications lost during the experimental processes. Nevertheless, the discrepancy between the masses detected by Orbitrap MS and those detected by the LCQ Advantage cannot be solely explained by adducts and artefacts and suggests that hundreds of peptides are present in the venom. *De novo* mass spectrometry of the venom of *T. bicarinatum* indicates that similar peptide processing occurred, supporting the idea that mature peptides could be cleaved in the venom. This is exemplified by the precursor prepro-U₁-MYRTX-Tbla which generates at least 32 peptides in the venom including part of the pro-peptide (Table S-4). Another example from our study is the peptide fragments found in the U₃-MYRTX group which seem to be preferentially cleaved around a methionine residue. However, it is remaining to known if these fragmented peptides which are likely related to peptide degradation, are functionally active.

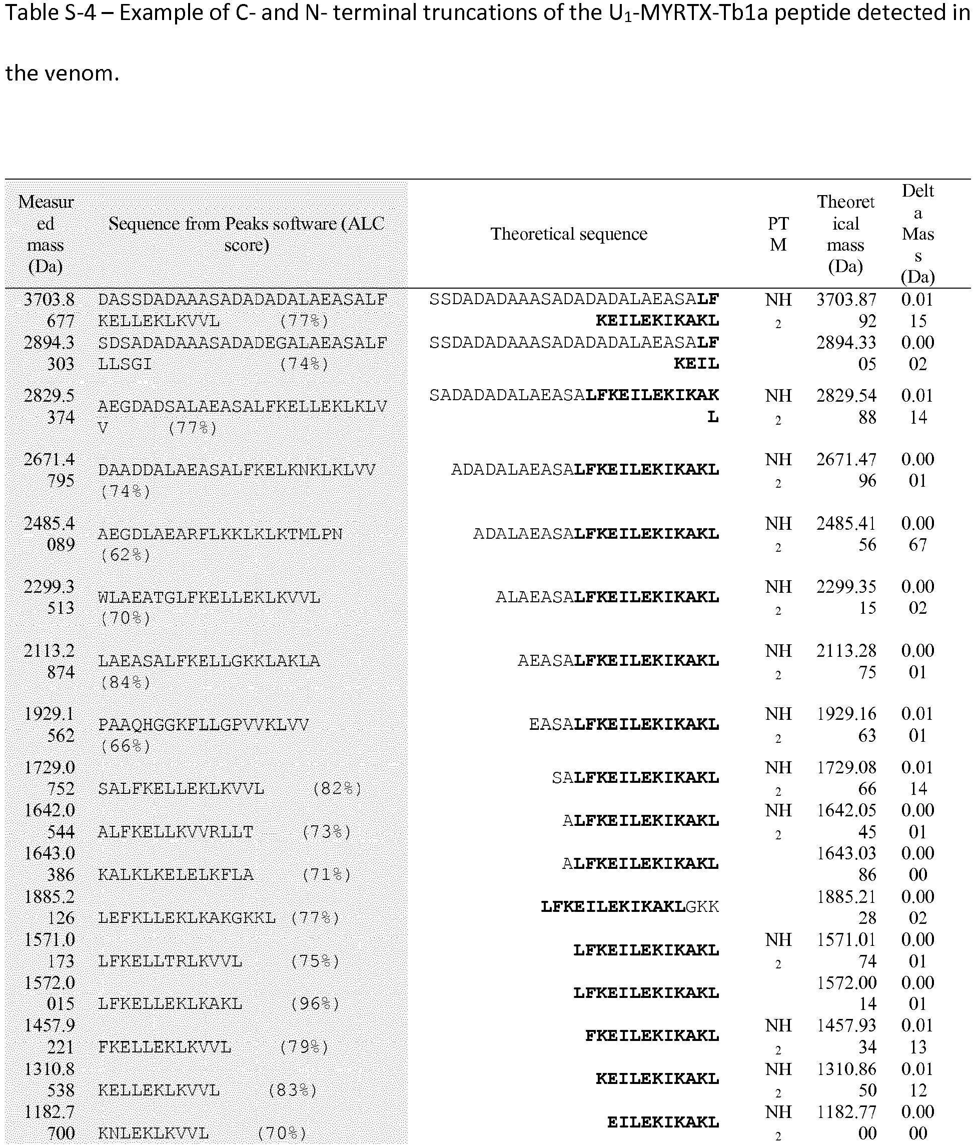

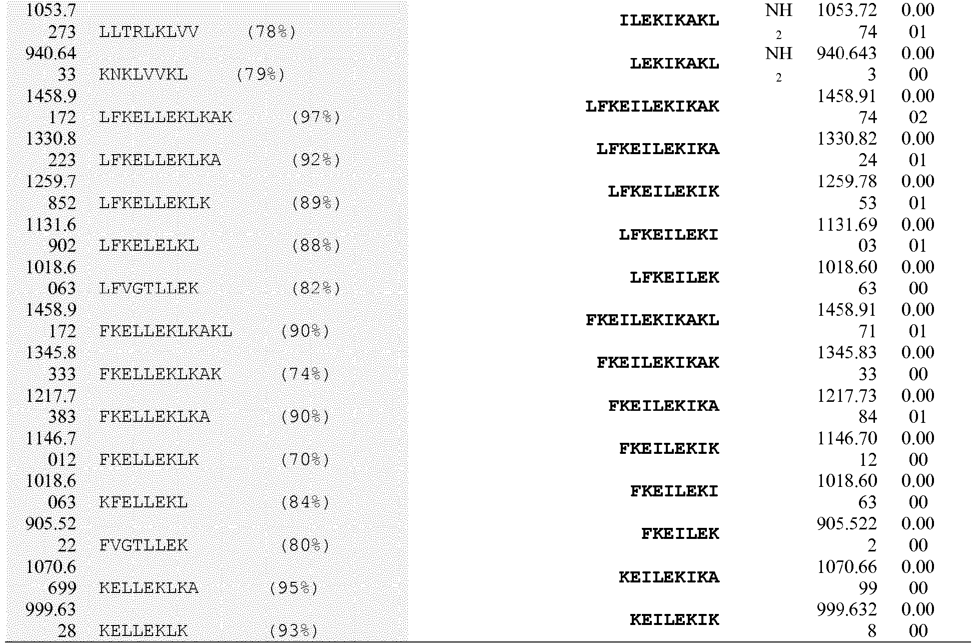

Bold residues indicate the mature sequence of U₁-MYRTX-Tb1a (P17) and shaded area show the sequences, masses and ALC scores from Peaks software. Note that I and L residues cannot be differentiated from the Peaks information.

### 4. CONCLUDING REMARKS

In summary, we provide the first insight into the whole peptide arsenal that composes an ant venom. The integrated methodology employed in this study has allowed the identification of 37 venom peptide precursors which are processed into several thousand peptides in the venom. Indeed, our mass spectrometry analysis revealed that peptide fragmentation and variable N- and C-terminal truncations generated a huge number of myrmicitoxins from a limited set of precursors. We also demonstrated that three myrmicitoxins were O-glycosylated on their N-terminal threonine residue. Most of the myrmicitoxins found in *T. bicarinatum* venom are presumably membrane-active peptides and may possess antimicrobial, cytolytic and insecticidal properties. It is also worth noting that *T. bicarinatum* venom glands expressed homologous toxins of the neurotoxic peptide δ-PPONTX-Pc1a (poneratoxin) only known from the ant *Paraponera clavata.* Nevertheless, future functional studies are required to confirm the biological activities of these myrmicitoxins. From an evolutionary perspective, our data suggest that several of the myrmicitoxins described here may have evolved from the innate immunity system of ants.

### Brief description of the Figures

**Figure** 1. Positive mode total ion chromatogram of crude venom of *T. bicarinatum* using LCQ ESI mass spectrometer. The venom was separated onto a C₁₈ column. The mobile phase was 0.1% aqueous formic acid (solvent A) and 0.1% formic acid in acetonitrile (solvent B). Peptides were eluted using a linear gradient from 0 to 50% B over 45 min, from 50 to 100% B over 10 minutes and then held for 5 min at 200 µL.min⁻¹ flow rate.
**Figure** 2. Multiple alignment of *T*. *bicarinatum* venom peptide precursors in superfamily-A, -B and -C. Alignments were generated with muscle on Seaview 1:4.5.4.8-2 and edited with Boxshade 3.3.1-9. Gaps were introduced to optimize the alignments. Pilosulin precursors from *Myrmecia pilosula* which have homology sequences with MYRTXs have been included. Identical residues are highlighted in magenta. Similar residues in the peptide sequences are boxed in blue while conserved residues are shown in cyan. Black triangles indicate the cleavage site releasing mature peptides. * : For prepro-U13-MYRTX-Tbla the cleavage site producing mature peptide occurs after the GEAEAEG motif. The predicted signal sequences are underlined in black.
**Figure 3****.** Multiple sequence alignment of mature myrmecitoxins from *Tetramorium bicarinatum* venom with homologous venom peptides from hymenoptera. Resulting alignments from ClustalX program were edited with Boxshade 3.3.1-9. Gaps were introduced to optimize the alignments. Identical residues are highlighted in magenta. Similar residues in the peptide sequences are boxed in blue while conserved residues are shown in cyan. Percentage identity (% ID) is relative to the first peptide of each family. Red stars represent C-terminal amidation and the threonine residues within the red box are O-glycosylated.
**Figure 4****.** Fragmentation patterns of HexNAc-threonine and in situ generated oxonium ions (A). Saccharide oxonium ions spectra of native U17-Tblc (Tb2853), U17-Tblb (Tb2774) and U17-Tbla (Tb2671) (B)
**Figure 5**. Minimum evolution phylogenetic trees of venom peptides precursors from *T*. *bicarinatum.* Alignments of prepropeptide regions were generated with Muscle on Seaview 1:4.5.4.8-2, then analyzed with MEGA 7.0.26 with minimum evolution statistical test and edited on Seaview 1:4.5.4.8-2. Precursors from *T. bicarinatum* are boxed in color. A: superfamily-A, pilosulin, other venom peptides and antimicrobial peptide precursors, **B** : superfamily-B and antimicrobial peptide precursors, **C:** superfamily-C and secapin precursors.
**Figure 6****.** Predicted maturation pathways of venom peptides precursors. A : superfamily-A, B : superfamily-B and C : superfamily-C (secapin). For superfamily-C, the example of U17-MYRTX-Tbla with O-glycosylation step on the N-terminal threonine, amidation at the C-terminus and one disulfide bond has been chosen. Two hypothesis of cleavage are presented: in the Golgi apparatus or at the membrane level.
   ? amidation, ● : cystein, 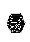 N-acetylgalactosamine. Threonine
**Figure 7****.** Polymorphism between nucleotide sequences encoding precursors of superfamily A (A) and B (B). Green positions indicate non synonymous substitutions that conserved the polarity of the amino acid, in red: non synonymous substitutions that change the polarity of amino acid, Light blue: hypervariable nucleotide sequences matching with the mature part of the precursor. Points indicate conserved nucleotide, dashes indicate gaps.
**Figure 8****.** Alignment of secapin precursor sequences from *Tetramorium bicarinatum* and others ants. Alignment was generated with muscle on Seaview 1:4.5.4.8-2 and edited with Boxshade 3.3.1-9. Gaps were introduced to optimize the alignments. Identical residues are highlighted in black. Similar residues in the peptide sequences are boxed in dark grey while conserved residues are shown in light grey.

## Claims

1. An isolated venom peptide precursor comprising a prepropeptide sequence selected from the group consisting of
- SEQ ID No. 95, more preferably SEQ ID No. 1,
- SEQ ID No. 96, more preferably SEQ ID No. 2,
- SEQ ID No. 3 or SEQ ID No.4
and a mature peptide.

2. The isolated venom peptide precursor of claim 1 wherein the prepropeptide sequence is SEQ ID No. 95, the cleavage motif between the prepropeptide and the mature peptide is SEQ ID No. 11.

3. The isolated venom peptide precursor of claim 1 ou 2 wherein the prepropeptide sequence is SEQ ID No. 1, the cleavage motif between the prepropeptide and the mature peptide is SEQ ID No. 11.

4. The isolated venom peptide precursor of claim 1 wherein the prepropeptide sequence is SEQ ID No. 96, the cleavage motif between the prepropeptide and the mature peptide is chosen among SEQ ID No. 12 to SEQ ID No. 15.

5. The isolated venom peptide precursor of claim 1 or 4, wherein the prepropeptide sequence is SEQ ID No. 2, the cleavage motif between the prepropeptide and the mature peptide is chosen among SEQ ID No. 12 to SEQ ID No. 15.

6. The isolated venom peptide precursor of claim 1 wherein the prepropeptide sequence SEQ ID No. 3 or SEQ ID No.4 the cleavage motif between the prepropeptide and the mature peptide is chosen among GEA, SEA, TEA, TEF or TEG.

7. The isolated venom peptide precursor of anyone of claims 1, 2 or 3 selected from SEQ ID No.16 to SEQ ID No. 30.

8. The isolated venom peptide precursor of anyone of claim 1, 2, 3 or 7 being a pilosulin precursor.

9. The isolated venom peptide precursor of anyone of claim 1, 4 or 5 selected from SEQ ID No.31 to SEQ ID No. 38.

10. The isolated venom peptide precursor of anyone of claim 1, 4, 5 or 9 being a antimicrobial precursor.

11. The isolated venom precursor of claim 1 or 6 selected from SEQ ID No.39 to SEQ ID No. 50.

12. The isolated venom precursor of anyone of claim 1, 6 or 11 being a secapin precursor.

13. The mature venom peptide deriving from an isolated venom precursor of anyone of preceding claim and chosen among SEQ ID No. 9, SEQ ID No.51 to SEQ ID No. 88.

14. A nucleic acid encoding anyone of peptide chosen among SEQ ID No. 16 to 88.


SEQUENCE LISTING

<110>  TTT

<120>  ANT VENOM PEPTIDOME

<130>  BR099803

<160>  96

<170>  PatentIn version 3.5

<210>  1
<211>  39
<212>  PRT
<213>  Tetramorium BICARINATUM



<220>
<221>  MISC_FEATURE
<223>  X : anyone of the 20 amino acids

<220>
<221>  misc_feature
<222>  (6)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (13)..(13)
<223>  Xaa can be any naturally occurring amino acid

<220>
<221>  misc_feature
<222>  (18)..(21)
<223>  Xaa can be any naturally occurring amino acid

<220>
<221>  misc_feature
<222>  (24)..(24)
<223>  Xaa can be any naturally occurring amino acid

<220>
<221>  misc_feature
<222>  (26)..(28)
<223>  Xaa can be any naturally occurring amino acid

<220>
<221>  misc_feature
<222>  (32)..(32)
<223>  Xaa can be any naturally occurring amino acid

<400>  1

Met Lys Val Leu Ser Xaa Leu Xaa Leu Ala Xaa Ala Xaa Ile Phe Val
1               5                   10                  15

Met Xaa Xaa Xaa Xaa Trp Ala Xaa Pro Xaa Xaa Xaa Ala Lys Ala Xaa
            20                  25                  30

Ala Asp Ala Asp Ala Asp Ala
        35

<210>  2
<211>  24
<212>  PRT
<213>  TETRAMORIUM BICARINATUM

<220>
<221>  MISC_FEATURE
<222>  (3)..(3)
<223>  X is I, L , S or no amino acid

<220>
<221>  MISC_FEATURE
<222>  (5)..(5)
<223>  X is K, N , Y or no amino acid

<220>
<221>  misc_feature
<222>  (18)..(18)
<223>  Xaa can be any naturally occurring amino acid

<220>
<221>  misc_feature
<222>  (20)..(21)
<223>  Xaa can be any naturally occurring amino acid

<400>  2

Met Lys Xaa Ile Xaa Leu Ile Thr Ile Phe Ala Met Ile Ala Thr Leu
1               5                   10                  15

Met Xaa Thr Xaa Xaa Gly Glu Ala
            20

<210>  3
<211>  33
<212>  PRT
<213>  TETRAMORIUM BICARINATUM

<220>
<221>  misc_feature
<222>  (7)..(7)
<223>  Xaa can be any naturally occurring amino acid

<220>
<221>  misc_feature
<222>  (11)..(11)

<223>  Xaa can be any naturally occurring amino acid

<220>
<221>  misc_feature
<222>  (14)..(14)
<223>  Xaa can be any naturally occurring amino acid

<220>
<221>  misc_feature
<222>  (17)..(18)
<223>  Xaa can be any naturally occurring amino acid

<220>
<221>  misc_feature
<222>  (33)..(33)
<223>  Xaa can be any naturally occurring amino acid

<400>  3

Met Glu Lys Asn Arg Thr Xaa Thr Phe Ser Xaa Tyr Leu Xaa Ile Thr
1               5                   10                  15

Xaa Xaa Leu Ile Ser Thr Phe Ile Thr Met Val Ile Thr Asp Gly Glu
            20                  25                  30

Xaa

<210>  4
<211>  31
<212>  PRT
<213>  TETRAMORIUM BICARINATUM

<220>
<221>  misc_feature
<222>  (7)..(7)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (14)..(14)
<223>  Xaa can be any naturally occurring amino acid

<220>
<221>  misc_feature
<222>  (17)..(18)
<223>  Xaa can be any naturally occurring amino acid

<220>
<221>  misc_feature
<222>  (31)..(31)
<223>  Xaa can be any naturally occurring amino acid

<400>  4

Met Glu Lys Asn Arg Thr Xaa Thr Phe Ser Xaa Tyr Leu Xaa Ile Thr
1               5                   10                  15

Xaa Xaa Leu Ile Ser Thr Phe Ile Thr Met Val Ile Thr Glu Xaa
            20                  25                  30

<210>  5
<211>  9
<212>  PRT
<213>  TETRAMORIUM BICARINATUM

<220>
<221>  MISC_FEATURE
<222>  (8)..(8)
<223>  X is A, S or G

<220>
<221>  MISC_FEATURE
<222>  (9)..(9)
<223>  X is A, S or G

<400>  5

Thr Met Val Ile Thr Asp Gly Glu Xaa
1               5

<210>  6
<211>  9
<212>  PRT
<213>  TETRAMORIUM BICARINATUM

<220>
<221>  MISC_FEATURE
<222>  (9)..(9)
<223>  X is A, S or G

<400>  6

Thr Met Val Met Ser Asp Gly Glu Xaa
1               5

<210>  7
<211>  7
<212>  PRT
<213>  TETRAMORIUM BICARINATUM

<220>
<221>  MISC_FEATURE
<222>  (7)..(7)
<223>  X is A, S or G

<400>  7

Thr Met Val Ile Thr Glu Xaa
1               5

<210>  8
<211>  7
<212>  PRT
<213>  TETRAMORIUM BICARINATUM

<220>
<221>  MISC_FEATURE
<222>  (7)..(7)
<223>  X is A, S or G

<400>  8

Thr Met Val Met Ser Glu Xaa
1               5

<210>  9
<211>  30
<212>  PRT
<213>  TETRAMORIUM BICARINATUM

<220>
<221>  misc_feature
<222>  (1)..(1)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (15)..(15)
<223>  Xaa can be any naturally occurring amino acid

<220>
<221>  misc_feature
<222>  (17)..(22)
<223>  Xaa can be any naturally occurring amino acid

<220>
<221>  misc_feature
<222>  (24)..(24)
<223>  Xaa can be any naturally occurring amino acid

<220>
<221>  misc_feature
<222>  (27)..(28)

<223>  Xaa can be any naturally occurring amino acid

<220>
<221>  misc_feature
<222>  (30)..(30)
<223>  Xaa can be any naturally occurring amino acid

<400>  9

Xaa Ile Ile Xaa Xaa Pro Xaa Phe Xaa Pro Pro Thr Cys Pro Xaa Gly
1               5                   10                  15


Xaa Xaa Xaa Xaa Xaa Xaa Gly Xaa Cys Arg Xaa Xaa Phe Xaa
            20                  25                  30


<210>  10
<211>  5
<212>  PRT
<213>  TETRAMORIUM BICARINATUM

<400>  10

Ala Asp Ala Asp Ala
1               5


<210>  11
<211>  4
<212>  PRT
<213>  TETRAMORIUM BICARINATUM


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<400>  11

Xaa Ala Xaa Ala
1


<210>  12
<211>  4
<212>  PRT
<213>  TETRAMORIUM BICARINATUM


<220>
<221>  misc_feature
<222>  (1)..(1)
<223>  Xaa can be any naturally occurring amino acid

<400>  12

Xaa Gly Glu Ala
1

<210> 13
<211> 4
<212> PRT
<213> TETRAMORIUM BICARINATUM

<400> 13

Asn Gly Glu Pro
1

<210> 14
<211> 4
<212> PRT
<213> TETRAMORIUM BICARINATUM

<400> 14

Glu Ala Glu Gly
1

<210> 15
<211> 4
<212> PRT
<213> TETRAMORIUM BICARINATUM

<400> 15

Asn Gly Lys Ala
1

<210> 16
<211> 46
<212> PRT
<213> TETRAMORIUM BICARINATUM

<400> 16

Met Gln Leu Ser His Leu Leu Leu Ala Phe Ala Met Ile Phe Val Met
1               5                   10                  15

Val Ile Thr Tyr Ala Pro Gln Val Gln Ala Asp Ala Met Ala Asp Ala
            20                  25                  30

Lys Arg Cys Glu Pro Asp Arg Ala Arg Arg Gly Leu Cys Gly
        35                  40                  45

<210> 17
<211> 56
<212> PRT
<213> TETRAMORIUM BICARINATUM

<400> 17

Met Gln Pro Ser Tyr Leu Leu Leu Ala Phe Ala Ile Ile Phe Val Met
1               5                   10                  15

Val Ile Met Tyr Ser Pro Ala Val Glu Ala Lys Ala Gly Ala Asp Ala
            20                  25                  30

Asp Ala Asp Ala His Ala Asp Ala Gly Cys Ser Gln Phe Arg Arg Met
        35                  40                  45

Arg Asn Leu Cys Gly Gly Lys Lys
    50                  55

<210>  18
<211>  54
<212>  PRT
<213>  TETRAMORIUM BICARINATUM

<400>  18

Met Gln Leu Ser His Leu Leu Leu Ala Phe Ala Met Ile Phe Val Met
1               5                   10                  15

Thr Ile Ile His Thr Pro Gln Val Gln Ala Asp Ala Met Ala Asp Ala
            20                  25                  30

Asp Ala Asp Ala Ala Ile Asn Cys Arg Arg Tyr Pro Arg His Pro Lys
        35                  40                  45

Cys Arg Gly Val Ser Ala
    50

<210>  19
<211>  66
<212>  PRT
<213>  TETRAMORIUM BICARINATUM

<400>  19

Met Gln Leu Ser His Leu Leu Leu Ala Phe Ala Met Ile Phe Val Met
1               5                   10                  15

Thr Val Met Tyr Ala Pro Gln Val Gln Ala Asp Ala Trp Ala Asp Ala
            20                  25                  30

Asn Ala Asp Ala Asp Val Asn Cys Glu Val Thr Pro Tyr His Pro Asp
        35                  40                  45

Cys Arg Gly Val Ser Ile Leu Pro Arg Arg Trp Thr Lys Ile Cys Tyr
    50                  55                  60

Arg Cys

65

<210>  20
<211>  43
<212>  PRT
<213>  TETRAMORIUM BICARINATUM

<400>  20

Glu Ala Met Ala Glu Ala Met Ala Asp Ala Met Ala Asp Ala Met Ala
1               5                   10                  15

Asp Ala Met Ala Asp Ala Met Ala Asp Ala Met Ala Glu Ala Ala Ala
            20                  25                  30

Asp Pro Pro Pro Gly Phe Ile Gly Val Arg Gly
        35                  40

<210>  21
<211>  72
<212>  PRT
<213>  TETRAMORIUM BICARINATUM

<400>  21

Met Lys Leu Ser Phe Leu Ser Leu Ala Leu Ala Ile Ile Phe Val Met
1               5                   10                  15

Ala Thr Ile Tyr Ala Pro Gln Val Lys Ala Lys Ala Ser Ala Asp Ala
            20                  25                  30

Asp Ala Asp Ala Asp Ala Ala Ala Ser Ala Asn Ala Leu Ala Glu Ala
        35                  40                  45

Ser Ala Gly Ile Val Thr Lys Leu Ile Lys Lys Gly Val Lys Leu Gly
    50                  55                  60

Leu Lys Met Ala Leu Gly Lys Lys
65                  70

<210>  22
<211>  66
<212>  PRT
<213>  TETRAMORIUM BICARINATUM

<400>  22

Met Lys Leu Ser Phe Leu Ser Leu Ala Phe Ala Val Ile Phe Val Met
1               5                   10                  15

Ala Ile Met Tyr Ala Pro Gln Val Glu Ala Lys Ala Ser Ala Asp Ala
            20                  25                  30

Asp Ala Asp Ala Asp Ala Ala Ala Ser Ala Asp Ala Leu Ala Lys Ala
        35                  40                  45

Ser Ala Gly Met Leu Asp Arg Ile Leu Gly Ala Val Lys Gly Phe Met
    50                  55                  60

Gly Ser
65

<210>  23
<211>  58
<212>  PRT
<213>  TETRAMORIUM BICARINATUM

<400>  23

Leu Ala Met Ala Met Gly Asp Ala Val Ala Asp Ala Gln Ala Arg Ala
1               5                   10                  15

Met Ala Ala Ala Tyr Ala Ile Ala Gly Lys Glu Lys Glu Lys Leu Lys
            20                  25                  30

Gln Cys Phe Lys Asp Met Thr Leu Ala Ala Ile Asp Tyr Ala Lys His
        35                  40                  45

Lys Val Glu Lys His Leu Phe Lys Cys Ile
    50                  55

<210>  24
<211>  70
<212>  PRT
<213>  TETRAMORIUM BICARINATUM

<400>  24

Met Arg Val Ser Tyr Leu Ser Leu Thr Leu Thr Ile Val Val Val Ile
1               5                   10                  15

Ala Ile Ile Pro Glu Thr Glu Ala Lys Ala Trp Ala Asp Ala Asp Ala
            20                  25                  30

Glu Ala Gly Leu Gly Phe Leu Ala Lys Ile Met Gly Lys Val Gly Met
        35                  40                  45

Arg Met Ile Lys Lys Leu Val Pro Glu Ala Ala Lys Val Ala Val Asp
    50                  55                  60

Gln Leu Ser Gln Gln Gln
65                  70

<210>  25
<211>  40
<212>  PRT
<213>  TETRAMORIUM BICARINATUM

<400>  25

Leu Ala Val Ile Val Thr Leu Ala Ile Thr Ile Val Pro Thr Val Tyr
1               5                   10                  15

Ala Asn Ala Glu Ala Asp Ala Asp Ala Leu Trp Gly Lys Cys Pro Lys
            20                  25                  30

Ile Gly Gly Arg Arg Val Met Cys
        35                  40

<210>  26
<211>  40
<212>  PRT
<213>  TETRAMORIUM BICARINATUM

<400>  26

Leu Ala Val Ile Val Thr Leu Ala Ile Thr Ile Val Pro Thr Val Tyr
1               5                   10                  15

Ala Asn Ala Glu Ala Asp Ala Asp Ala Leu Trp Gly Lys Cys Pro Lys
            20                  25                  30

Ile Gly Gly Arg Arg Ile Met Cys
        35                  40

<210>  27
<211>  49
<212>  PRT
<213>  TETRAMORIUM BICARINATUM

<400>  27

Met Leu Gln Ala Leu Cys Phe Ser Leu Leu Ala Thr Ile Val Thr Leu
1               5                   10                  15

Thr Ile Ile Ile Met Pro Ala Ala Tyr Ala Asn Ala Glu Pro Glu Ala
            20                  25                  30

Phe Arg Gly Pro Cys Pro Lys Asp Met Phe Lys Gly Arg Phe Ile Met
        35                  40                  45

Cys

<210>  28
<211>  73

<212>  PRT
<213>  TETRAMORIUM BICARINATUM

<400>  28

Met Lys Val Leu Lys Phe Leu Phe Ile Ala Val Ile Ile Val Gly Leu
1               5                   10                  15

Ser Gly Ser Leu Thr Trp Ala Ser Pro Ile Ala Asn Ala Arg Ala Glu
            20                  25                  30

Ala Asp Ala Glu Ala Ala Ala Glu Ala Val Ala Lys Ala Val Ala Glu
        35                  40                  45

Ala Val Leu Pro Ala Leu Pro Leu Leu Ala Gly Leu Met Ser Leu Pro
    50                  55                  60

Phe Leu Gln His Lys Leu Thr Asn Gly
65                  70

<210>  29
<211>  73
<212>  PRT
<213>  TETRAMORIUM BICARINATUM

<400>  29

Met Lys Val Leu Lys Phe Leu Phe Ile Ala Val Ile Ile Val Gly Leu
1               5                   10                  15

Ser Gly Ser Leu Thr Trp Ala Ser Pro Leu Ala Lys Ala Lys Ala Glu
            20                  25                  30

Ala Asp Ala Met Ala Ala Ala Glu Ala Ile Ala Pro Ile Leu Ala Leu
        35                  40                  45

Pro Leu Leu Gly Gly Met Met Ser Leu Pro Phe Leu His His Lys Leu
    50                  55                  60

Thr Gly Gly Lys Pro His His Glu Gly
65                  70

<210>  30
<211>  81
<212>  PRT
<213>  TETRAMORIUM BICARINATUM

<400>  30

Met Lys Val Leu Lys Phe Leu Phe Ile Ala Val Ile Ile Val Gly Leu
1               5                   10                  15

Ser Gly Ser Leu Thr Trp Ala Ser Pro Leu Ala Lys Ala Lys Ala Glu
                20                  25                  30

Ala Asp Ala Met Ala Ala Ala Glu Ala Leu Ala Glu Ala Glu Pro Glu
        35                  40                  45

Ala Ile Ala Pro Ile Val Ala Leu Leu Leu Leu Ser Gly Leu Phe Ser
    50                  55                  60

Leu Pro Phe Leu His His Lys Leu Thr Asn Gly Thr Met His His Glu
65                  70                  75                  80

Gly

<210>  31
<211>  36
<212>  PRT
<213>  TETRAMORIUM BICARINATUM

<400>  31

Met Lys Ile Val Lys Leu Ile Thr Ile Phe Ala Met Ile Ala Thr Leu
1               5                   10                  15

Met Val Ala Val Ala Asn Gly Lys Ala Leu Ser Pro Ala Val Leu Ala
                20                  25                  30

Ser Leu Ala Gly
        35

<210>  32
<211>  36
<212>  PRT
<213>  TETRAMORIUM BICARINATUM

<400>  32

Met Lys Ile Ile Lys Leu Ile Thr Ile Phe Thr Met Met Ala Thr Leu
1               5                   10                  15

Met Val Ala Val Ala Asn Gly Glu Pro Ile Pro Pro Asn Ala Val Lys
                20                  25                  30

Ser Leu Gln Gly
        35

<210>  33
<211>  41
<212>  PRT
<213>  TETRAMORIUM BICARINATUM

<400>  33

Met Lys Ile Val Lys Leu Ile Thr Ile Phe Ala Met Ile Ala Thr Leu
1               5                   10                  15

Met Val Ala Val Ala Asn Gly Glu Ala Val Phe Leu Thr Pro Asp Gln
            20                  25                  30

Ile Lys Ala Met Ile Lys Arg His Gly
        35                  40

<210>  34
<211>  40
<212>  PRT
<213>  TETRAMORIUM BICARINATUM

<400>  34

Met Lys Ile Val Lys Leu Ile Thr Ile Phe Ala Met Ile Ala Thr Leu
1               5                   10                  15

Met Val Ala Val Ala Asn Gly Glu Ala Ile Leu Thr Ala Asp Gln Leu
            20                  25                  30

Lys Ala Ile Ile Lys Arg His Gly
        35                  40

<210>  35
<211>  38
<212>  PRT
<213>  TETRAMORIUM BICARINATUM

<400>  35

Met Lys Ser Ile Asn Leu Ile Thr Ile Phe Ala Met Ile Ala Thr Leu
1               5                   10                  15

Met Ala Thr Met Ile Ala Gly Glu Ala Ala Arg Ser Arg Leu Lys Ile
            20                  25                  30

Gly Arg Met Gly Arg Lys
        35

<210>  36
<211>  45
<212>  PRT
<213>  TETRAMORIUM BICARINATUM

<400>  36

Met Lys Ser Ile Asn Leu Ile Thr Ile Phe Ala Met Ile Ala Thr Leu
1               5                   10                  15

Met Ala Thr Met Ile Ala Gly Glu Ala Ala Arg Val Ser Asp Cys Thr
            20                  25                  30

Met Phe Thr Ser Lys Leu Lys Leu Lys Leu Ile Pro Val
        35                  40                  45

<210>  37
<211>  53
<212>  PRT
<213>  TETRAMORIUM BICARINATUM

<400>  37

Met Lys Leu Ile Tyr Ile Phe Leu Leu Val Ala Val Ile Ala Val Thr
1               5                   10                  15

Met Ile Pro Gly Ile Met Gly Glu Ala Gly Asp Glu Ala Gly Lys Pro
            20                  25                  30

Lys Ile Gly Val Phe His Asp Val Asn Lys Ala Ile Glu Trp Leu Leu
        35                  40                  45

Lys Gln Thr Lys Gly
    50

<210>  38
<211>  53
<212>  PRT
<213>  TETRAMORIUM BICARINATUM

<400>  38

Met Lys Leu Ile Tyr Ile Phe Ser Leu Val Ala Val Val Ile Val Thr
1               5                   10                  15

Met Ile Pro Gly Ile Met Gly Glu Ala Glu Ala Glu Gly Arg Pro Pro
            20                  25                  30

Gln Ile Gly Ile Phe Asp Gln Ile Asp Lys Gly Met Ala Ala Phe Met
        35                  40                  45

Asp Leu Phe Lys Gly
    50

<210>  39
<211>  57
<212>  PRT
<213>  TETRAMORIUM BICARINATUM

<400>  39

Met Glu Lys Asn Arg Thr Asn Ile Phe Ser Val Tyr Leu Met Ile Thr
1               5                   10                  15

Phe Leu Leu Ile Ser Ile Phe Ile Thr Met Val Met Ser Asp Gly Glu
            20                  25                  30

Ala Thr Ile Ile Asn Ala Pro Asn Arg Cys Pro Pro Gly His Val Val
        35                  40                  45

Val Lys Gly Arg Cys Arg Ile Ala Gly
    50                  55

<210>  40
<211>  57
<212>  PRT
<213>  TETRAMORIUM BICARINATUM

<400>  40

Met Glu Lys Asn Arg Thr Asn Ile Phe Ser Val Tyr Leu Met Ile Thr
1               5                   10                  15

Phe Leu Leu Ile Ser Ile Phe Ile Thr Met Val Met Ser Asp Ser Glu
            20                  25                  30

Ala Thr Val Ile Asp Val Pro Ile Gln Cys Pro Ser Gly Thr Val Lys
        35                  40                  45

Val Gly Asn Lys Cys Arg Val Ile Phe
    50                  55

<210>  41
<211>  57
<212>  PRT
<213>  TETRAMORIUM BICARINATUM

<400>  41

Met Glu Lys His Arg Thr Asn Ile Phe Ser Val Tyr Leu Met Ile Thr
1               5                   10                  15

Phe Leu Leu Ile Ser Ile Phe Ile Thr Met Val Met Ser Asp Gly Glu
            20                  25                  30

Ala Thr Ile Ile Asp Val Pro Ile Gln Cys Pro Pro Gly Lys Val Lys
        35                  40                  45

Val Gly Asn Arg Cys Arg Val Ile Phe
    50                  55

<210>  42
<211>  55
<212>  PRT

<213>  TETRAMORIUM BICARINATUM

<400>  42

Met Glu Lys Asn Arg Thr Thr Thr Phe Ser Val Tyr Leu Thr Ile Ile
1               5                   10                  15

Leu Phe Leu Ile Ser Thr Phe Ile Thr Met Val Ile Thr Glu Ala Asn
            20                  25                  30

Ile Ile Arg Val Pro Cys Arg Ala Gly Tyr Ile Glu Val Asn Gly Val
        35                  40                  45

Cys Arg Glu Val Phe Thr Gly
    50                  55

<210>  43
<211>  59
<212>  PRT
<213>  TETRAMORIUM BICARINATUM

<400>  43

Met Glu Lys Asn Arg Thr Thr Thr Phe Ser Val Tyr Leu Thr Ile Thr
1               5                   10                  15

Leu Phe Leu Ile Ser Thr Phe Ile Thr Met Val Ile Thr Glu Ser Asn
            20                  25                  30

Ile Ile Lys Ala Pro Leu Phe Pro Cys Pro Asn Gly Tyr Ile Arg Asp
        35                  40                  45

Tyr Lys Gly Asp Cys Arg Glu Ile Ile Glu Gly
    50                  55

<210>  44
<211>  59
<212>  PRT
<213>  TETRAMORIUM BICARINATUM

<400>  44

Met Glu Lys Asn Arg Thr Thr Thr Phe Ser Val Tyr Leu Thr Ile Ile
1               5                   10                  15

Leu Phe Leu Ile Ser Thr Phe Ile Thr Met Val Ile Thr Glu Ser Asn
            20                  25                  30

Ile Ile Arg Val Pro Glu Phe Gln Cys Pro Asn Gly Tyr Arg Lys Asp
        35                  40                  45

Ala Asn Gly Lys Cys Arg Glu Val Phe His Gly

50                  55

<210>  45
<211>  56
<212>  PRT
<213>  TETRAMORIUM BICARINATUM

<400>  45

Met Glu Lys Ser Arg Thr Gly Thr Phe Ser Ile Tyr Leu Met Ile Thr
1               5                   10                  15

Phe Leu Leu Ile Ser Thr Phe Ile Thr Met Val Ile Thr Glu Ala His
            20                  25                  30

Val Ile Asp Thr Arg Ser Arg Leu Cys Pro Glu Gly Ser Arg Arg Ser
        35                  40                  45

Thr Thr Gly Glu Cys Lys Thr Val
    50                  55

<210>  46
<211>  54
<212>  PRT
<213>  TETRAMORIUM BICARINATUM

<400>  46

Met Glu Lys Arg Val Ser Thr Phe Ser Ile Tyr Leu Ile Ile Thr Val
1               5                   10                  15

Phe Leu Ile Ser Thr Phe Ile Thr Met Val Ile Thr Glu Ala His Ile
            20                  25                  30

Ile Arg Val Pro Cys Arg Ala Gly Tyr Lys Glu Ile Arg Gly Arg Cys
        35                  40                  45

Arg Lys Ile Leu Thr Gly
    50

<210>  47
<211>  58
<212>  PRT
<213>  TETRAMORIUM BICARINATUM

<400>  47

Met Glu Lys Ser Pro Thr Thr Thr Phe Ser Ile Tyr Leu Met Thr Ile
1               5                   10                  15

Phe Leu Leu Ile Ser Thr Phe Ile Thr Met Val Ile Thr Glu Ser Arg
            20                  25                  30

Val Ile Asp Ala Arg Glu Arg Cys Pro Ser Gly Tyr Gln Met Asp Gly
        35                  40                  45

Ser Gly Lys Cys Arg Lys Ile Phe Gly Arg
    50                  55

<210>  48
<211>  60
<212>  PRT
<213>  TETRAMORIUM BICARINATUM

<400>  48

Met Gly Lys Asn Arg Thr Asn Ile Phe Ser Ile His Leu Met Ile Thr
1               5                   10                  15

Phe Leu Ile Ile Ser Ile Phe Phe Ile Thr Met Val Ile Ser Glu Ser
            20                  25                  30

Tyr Ile Ile Arg Val Pro Thr Phe Pro Pro Pro Thr Cys Pro Pro Gly
        35                  40                  45

Glu Thr Met Val Gly Lys Arg Cys Arg His Val Tyr
    50                  55                  60

<210>  49
<211>  59
<212>  PRT
<213>  TETRAMORIUM BICARINATUM

<400>  49

Met Glu Lys Asn Arg Thr Thr Thr Phe Ser Val Tyr Leu Thr Ile Thr
1               5                   10                  15

Leu Phe Leu Ile Ser Thr Phe Ile Thr Met Val Ile Thr Glu Ser Tyr
            20                  25                  30

Ile Ile Glu Ala Pro Pro Phe Pro Cys Pro Asn Gly Tyr Met Arg Asp
        35                  40                  45

Tyr Glu Gly Asp Cys Arg Glu Ile Phe Glu Gly
    50                  55

<210>  50
<211>  61
<212>  PRT
<213>  TETRAMORIUM BICARINATUM

<400>  50

Met Glu Lys Ser Arg Thr Ser Thr Phe Ser Ile Tyr Leu Met Ile Thr

1               5                   10                  15

Phe Leu Leu Val Ser Thr Phe Val Thr Met Val Ile Thr Glu Gly Asp
            20                  25                  30

Ile Ile Asp Val Pro Leu Arg Val Ser Lys Cys Pro Glu Gly Ser Arg
        35                  40                  45

Met Ser Ile Ile Gly Gln Cys Arg Lys Val Ser Lys Arg
    50                  55                  60

<210>  51
<211>  10
<212>  PRT
<213>  TETRAMORIUM BICARINATUM

<400>  51

Asp Pro Pro Pro Gly Phe Ile Gly Val Arg
1               5                   10

<210>  52
<211>  23
<212>  PRT
<213>  TETRAMORIUM BICARINATUM

<400>  52

Val Leu Pro Ala Leu Pro Leu Leu Ala Gly Leu Met Ser Leu Pro Phe
1               5                   10                  15

Leu Gln His Lys Leu Thr Asn
            20

<210>  53
<211>  12
<212>  PRT
<213>  TETRAMORIUM BICARINATUM

<400>  53

Val Leu Pro Ala Leu Pro Leu Leu Ala Gly Leu Met
1               5                   10

<210>  54
<211>  11
<212>  PRT
<213>  TETRAMORIUM BICARINATUM

<400>  54

Val Leu Pro Ala Leu Pro Leu Leu Ala Gly Leu
1               5                   10

<210>  55
<211>  12
<212>  PRT
<213>  TETRAMORIUM BICARINATUM

<400>  55

Met Ser Leu Pro Phe Leu Gln His Lys Leu Thr Asn
1               5                   10


<210>  56
<211>  11
<212>  PRT
<213>  TETRAMORIUM BICARINATUM

<400>  56

Ser Leu Pro Phe Leu Gln His Lys Leu Thr Asn
1               5                   10


<210>  57
<211>  31
<212>  PRT
<213>  TETRAMORIUM BICARINATUM

<400>  57

Ile Ala Pro Ile Leu Ala Leu Pro Leu Leu Gly Gly Met Met Ser Leu
1               5                   10                  15


Pro Phe Leu His His Lys Leu Thr Gly Gly Lys Pro His His Glu
            20                  25                  30


<210>  58
<211>  17
<212>  PRT
<213>  TETRAMORIUM BICARINATUM

<400>  58

Ser Leu Pro Phe Leu His His Lys Leu Thr Gly Gly Lys Pro His His
1               5                   10                  15


Glu



<210>  59
<211>  14
<212>  PRT
<213>  TETRAMORIUM BICARINATUM

<400>  59

Ile Ala Pro Ile Leu Ala Leu Pro Leu Leu Gly Gly Met Met
1               5                   10

<210>  60
<211>  13
<212>  PRT
<213>  TETRAMORIUM BICARINATUM

<400>  60

Gly Cys Ser Gln Phe Arg Arg Met Arg Asn Leu Cys Gly
1               5                   10


<210>  61
<211>  13
<212>  PRT
<213>  TETRAMORIUM BICARINATUM

<400>  61

Lys Arg Cys Glu Pro Asp Arg Ala Arg Arg Gly Leu Cys
1               5                   10


<210>  62
<211>  15
<212>  PRT
<213>  TETRAMORIUM BICARINATUM

<400>  62

Leu Trp Gly Lys Cys Pro Lys Ile Gly Gly Arg Arg Val Met Cys
1               5                   10                  15


<210>  63
<211>  15
<212>  PRT
<213>  TETRAMORIUM BICARINATUM

<400>  63

Leu Trp Gly Lys Cys Pro Lys Ile Gly Gly Arg Arg Ile Met Cys
1               5                   10                  15


<210>  64
<211>  17
<212>  PRT
<213>  TETRAMORIUM BICARINATUM

<400>  64

Phe Arg Gly Pro Cys Pro Lys Asp Met Phe Lys Gly Arg Phe Ile Met
1               5                   10                  15


Cys


<210>  65
<211>  18
<212>  PRT
<213>  TETRAMORIUM BICARINATUM

<400>  65

Ala Ile Asn Cys Arg Arg Tyr Pro Arg His Pro Lys Cys Arg Gly Val
1               5                   10                  15

Ser Ala

<210>  66
<211>  16
<212>  PRT
<213>  TETRAMORIUM BICARINATUM

<400>  66

Gly Met Leu Asp Arg Ile Leu Gly Ala Val Lys Gly Phe Met Gly Ser
1               5                   10                  15

<210>  67
<211>  19
<212>  PRT
<213>  TETRAMORIUM BICARINATUM

<400>  67

Gly Ile Val Thr Lys Leu Ile Lys Lys Gly Val Lys Leu Gly Leu Lys
1               5                   10                  15

Met Ala Leu

<210>  68
<211>  36
<212>  PRT
<213>  TETRAMORIUM BICARINATUM

<400>  68

Gly Leu Gly Phe Leu Ala Lys Ile Met Gly Lys Val Gly Met Arg Met
1               5                   10                  15

Ile Lys Lys Leu Val Pro Glu Ala Ala Lys Val Ala Val Asp Gln Leu
            20                  25                  30

Ser Gln Gln Gln
        35

<210>  69
<211>  21
<212>  PRT
<213>  TETRAMORIUM BICARINATUM

<400>  69

Met Ile Lys Lys Leu Val Pro Glu Ala Ala Lys Val Ala Val Asp Gln
1               5                   10                  15

Leu Ser Gln Gln Gln
            20

<210>  70
<211>  34
<212>  PRT
<213>  TETRAMORIUM BICARINATUM

<400>  70

Gly Lys Glu Lys Glu Lys Leu Lys Gln Cys Phe Lys Asp Met Thr Leu
1               5                   10                  15

Ala Ala Ile Asp Tyr Ala Lys His Lys Val Glu Lys His Leu Phe Lys
            20                  25                  30

Cys Ile

<210>  71
<211>  10
<212>  PRT
<213>  TETRAMORIUM BICARINATUM

<400>  71

Leu Ser Pro Ala Val Leu Ala Ser Leu Ala
1               5                   10

<210>  72
<211>  23
<212>  PRT
<213>  TETRAMORIUM BICARINATUM

<400>  72

Arg Pro Pro Gln Ile Gly Ile Phe Asp Gln Ile Asp Lys Gly Met Ala
1               5                   10                  15

Ala Phe Met Asp Leu Phe Lys
            20

<210>  73
<211>  18
<212>  PRT
<213>  TETRAMORIUM BICARINATUM

<400>  73

Arg Pro Pro Gln Ile Gly Ile Phe Asp Gln Ile Asp Lys Gly Met Ala
1               5                   10                  15

Ala Phe

<210> 74
<211> 10
<212> PRT
<213> TETRAMORIUM BICARINATUM

<400> 74

Ile Pro Pro Asn Ala Val Lys Ser Leu Gln
1               5                   10

<210> 75
<211> 14
<212> PRT
<213> TETRAMORIUM BICARINATUM

<400> 75

Ile Leu Thr Ala Asp Gln Leu Lys Ala Ile Ile Lys Arg His
1               5                   10

<210> 76
<211> 15
<212> PRT
<213> TETRAMORIUM BICARINATUM

<400> 76

Val Phe Leu Thr Pro Asp Gln Ile Lys Ala Met Ile Lys Arg His
1               5                   10                  15

<210> 77
<211> 27
<212> PRT
<213> TETRAMORIUM BICARINATUM

<400> 77

Gly Asp Glu Ala Gly Lys Pro Lys Ile Gly Val Phe His Asp Val Asn
1               5                   10                  15

Lys Ala Ile Glu Trp Leu Leu Lys Gln Thr Lys
            20                  25

<210> 78
<211> 22
<212> PRT
<213> TETRAMORIUM BICARINATUM

<400> 78

Gly Asp Glu Ala Gly Lys Pro Lys Ile Gly Val Phe His Asp Val Asn
1               5                   10                  15

Lys Ala Ile Glu Trp Leu
            20

<210>  79
<211>  15
<212>  PRT
<213>  TETRAMORIUM BICARINATUM

<400>  79

His Asp Val Asn Lys Ala Ile Glu Trp Leu Leu Lys Gln Thr Lys
1               5                   10                  15

<210>  80
<211>  23
<212>  PRT
<213>  TETRAMORIUM BICARINATUM

<400>  80

Thr Ile Ile Asn Ala Pro Asn Arg Cys Pro Pro Gly His Val Val Val
1               5                   10                  15

Lys Gly Arg Cys Arg Ile Ala
            20

<210>  81
<211>  24
<212>  PRT
<213>  TETRAMORIUM BICARINATUM

<400>  81

Thr Val Ile Asp Val Pro Ile Gln Cys Pro Ser Gly Thr Val Lys Val
1               5                   10                  15

Gly Asn Lys Cys Arg Val Ile Phe
            20

<210>  82
<211>  24
<212>  PRT
<213>  TETRAMORIUM BICARINATUM

<400>  82

Thr Ile Ile Asp Val Pro Ile Gln Cys Pro Pro Gly Lys Val Lys Val
1               5                   10                  15

Gly Asn Arg Cys Arg Val Ile Phe
            20

<210>  83
<211>  23

<212>  PRT
<213>  TETRAMORIUM BICARINATUM

<400>  83

Asn Ile Ile Arg Val Pro Cys Arg Ala Gly Tyr Ile Glu Val Asn Gly
1               5                   10                  15

Val Cys Arg Glu Val Phe Thr
            20

<210>  84
<211>  27
<212>  PRT
<213>  TETRAMORIUM BICARINATUM

<400>  84

Asn Ile Ile Lys Ala Pro Leu Phe Pro Cys Pro Asn Gly Tyr Ile Arg
1               5                   10                  15

Asp Tyr Lys Gly Asp Cys Arg Glu Ile Ile Glu
            20                  25

<210>  85
<211>  27
<212>  PRT
<213>  TETRAMORIUM BICARINATUM

<400>  85

Asn Ile Ile Arg Val Pro Glu Phe Gln Cys Pro Asn Gly Tyr Arg Lys
1               5                   10                  15

Asp Ala Asn Gly Lys Cys Arg Glu Val Phe His
            20                  25

<210>  86
<211>  26
<212>  PRT
<213>  TETRAMORIUM BICARINATUM

<400>  86

Ile Ile Arg Val Pro Glu Phe Gln Cys Pro Asn Gly Tyr Arg Lys Asp
1               5                   10                  15

Ala Asn Gly Lys Cys Arg Glu Val Phe His
            20                  25

<210>  87
<211>  25
<212>  PRT
<213>  TETRAMORIUM BICARINATUM

<400>  87

His Val Ile Asp Thr Arg Ser Arg Leu Cys Pro Glu Gly Ser Arg Arg
1               5                   10                  15

Ser Thr Thr Gly Glu Cys Lys Thr Val
            20                  25

<210>  88
<211>  23
<212>  PRT
<213>  TETRAMORIUM BICARINATUM

<400>  88

His Ile Ile Arg Val Pro Cys Arg Ala Gly Tyr Lys Glu Ile Arg Gly
1               5                   10                  15

Arg Cys Arg Lys Ile Leu Thr
            20

<210>  89
<211>  7
<212>  PRT
<213>  TETRAMORIUM BICARINATUM

<220>
<221>  misc_feature
<222>  (6)..(6)
<223>  Xaa can be any naturally occurring amino acid

<400>  89

Pro Pro Thr Cys Pro Xaa Gly
1               5

<210>  90
<211>  4
<212>  PRT
<213>  TETRAMORIUM BICARINATUM

<220>
<221>  misc_feature
<222>  (2)..(2)
<223>  Xaa can be any naturally occurring amino acid

<400>  90

Gly Xaa Cys Arg
1

<210>  91
<211>  79
<212>  PRT
<213>  TETRAMORIUM BICARINATUM

<400>  91

Met Lys Leu Ser Phe Leu Ser Leu Val Leu Ala Ile Ile Leu Val Met
1               5                   10                  15

Ala Leu Met Tyr Thr Pro His Ala Glu Ala Lys Ala Trp Ala Asp Ala
            20                  25                  30

Asp Ala Asp Ala Thr Ala Ala Ala Asp Ala Asp Ala Asp Ala Val Ala
        35                  40                  45

Asp Ala Leu Ala Asp Ala Val Ala Lys Ile Lys Ile Pro Trp Gly Lys
    50                  55                  60

Val Lys Asp Phe Leu Val Gly Gly Met Lys Ala Val Gly Lys Lys
65                  70                  75

<210>  92
<211>  68
<212>  PRT
<213>  TETRAMORIUM BICARINATUM

<400>  92

Met Lys Leu Ser Phe Leu Ser Leu Ala Leu Ala Thr Ile Phe Val Met
1               5                   10                  15

Ala Ile Ile Tyr Ala Pro Gln Met Glu Ala Arg Ala Ser Ser Asp Ala
            20                  25                  30

Asp Ala Asp Ala Ala Ala Ser Ala Asp Ala Asp Ala Asp Ala Leu Ala
        35                  40                  45

Glu Ala Ser Ala Leu Phe Lys Glu Ile Leu Glu Lys Ile Lys Ala Lys
    50                  55                  60

Leu Gly Lys Lys
65

<210>  93
<211>  20
<212>  PRT
<213>  TETRAMORIUM BICARINATUM

<400>  93

Lys Ile Lys Ile Pro Trp Gly Lys Val Lys Asp Phe Leu Val Gly Gly
1               5                   10                  15

Met Lys Ala Val
            20

<210>  94
<211>  13
<212>  PRT
<213>  TETRAMORIUM BICARINATUM

<400>  94

Leu Phe Lys Glu Ile Leu Glu Lys Ile Lys Ala Lys Leu
1               5                   10


<210>  95
<211>  59
<212>  PRT
<213>  TETRAMORIUM BICARINATUM


<220>
<221>  misc_feature
<222>  (3)..(3)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (13)..(13)
<223>  Xaa can be any naturally occurring amino acid

<220>
<221>  misc_feature
<222>  (15)..(15)
<223>  Xaa can be any naturally occurring amino acid

<220>
<221>  misc_feature
<222>  (18)..(21)
<223>  Xaa can be any naturally occurring amino acid

<220>
<221>  misc_feature
<222>  (24)..(24)
<223>  Xaa can be any naturally occurring amino acid

<220>
<221>  misc_feature
<222>  (26)..(28)
<223>  Xaa can be any naturally occurring amino acid

<220>
<221>  misc_feature
<222>  (30)..(30)
<223>  Xaa can be any naturally occurring amino acid

<220>
<221>  misc_feature
<222>  (32)..(32)
<223>  Xaa can be any naturally occurring amino acid

<220>
<221>  misc_feature
<222>  (40)..(40)
<223>  Xaa can be any naturally occurring amino acid

<220>
<221>  misc_feature
<222>  (42)..(42)
<223>  Xaa can be any naturally occurring amino acid

<220>
<221>  misc_feature
<222>  (44)..(44)
<223>  Xaa can be any naturally occurring amino acid

<220>
<221>  misc_feature
<222>  (46)..(46)
<223>  Xaa can be any naturally occurring amino acid

<220>
<221>  misc_feature
<222>  (48)..(48)
<223>  Xaa can be any naturally occurring amino acid

<220>
<221>  misc_feature
<222>  (50)..(50)
<223>  Xaa can be any naturally occurring amino acid

<220>
<221>  misc_feature
<222>  (52)..(52)
<223>  Xaa can be any naturally occurring amino acid

<220>
<221>  misc_feature
<222>  (54)..(54)
<223>  Xaa can be any naturally occurring amino acid

<400>  95

Met Lys Xaa Leu Ser Xaa Leu Xaa Leu Ala Xaa Ala Xaa Ile Xaa Val
1               5                   10                  15

Met Xaa Xaa Xaa Xaa Trp Ala Xaa Pro Xaa Xaa Xaa Ala Xaa Ala Xaa
            20                  25                  30

Ala Asp Ala Asp Ala Asp Ala Xaa Ala Xaa Ala Xaa Ala Xaa Ala Xaa
        35                  40                  45

Ala Xaa Ala Xaa Ala Xaa Ala Asp Ala Val Ala
    50                  55

<210>  96
<211>  25
<212>  PRT
<213>  TETRAMORIUM BICARINATUM

<220>
<221>  misc_feature
<222>  (3)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (18)..(18)
<223>  Xaa can be any naturally occurring amino acid

<220>
<221>  misc_feature
<222>  (20)..(22)
<223>  Xaa can be any naturally occurring amino acid

<400>  96

Met Lys Xaa Ile Xaa Leu Ile Thr Ile Phe Ala Met Ile Ala Thr Leu
1               5                   10                  15

Met Xaa Thr Xaa Xaa Xaa Gly Glu Ala
            20                  25